(19) Europäisches Patentamt
European Patent Office
Office européen des brevets

(11) Publication number: **0 648 759 A1**

(12) # EUROPEAN PATENT APPLICATION

(21) Application number: **94115981.6**

(51) Int. Cl.⁶: **C07D 401/10**, A61K 31/445

(22) Date of filing: **11.10.94**

(30) Priority: **15.10.93 IT MI932193**

(43) Date of publication of application:
**19.04.95 Bulletin 95/16**

(84) Designated Contracting States:
**AT BE CH DE DK ES FR GB GR IE IT LI LU MC NL PT SE**

(71) Applicant: **ERREGIERRE S.p.A.**
**Via Montenapoleone, 27/E**
**I-21021 Milan (IT)**

(72) Inventor: **Pellicciari Roberto**
**Via Ulisse Rocchi 60**
**I-06100 Perugia (IT)**

(74) Representative: **Gervasi, Gemma, Dr. et al**
**NOTARBARTOLO & GERVASI Srl**
**Viale Bianca Maria 33**
**I-20122 Milano (IT)**

(54) **Antihistaminic compounds, process for their preparation and pharmaceutical compositions containing them.**

(57) The present invention relates to terfenadine derivatives of formula

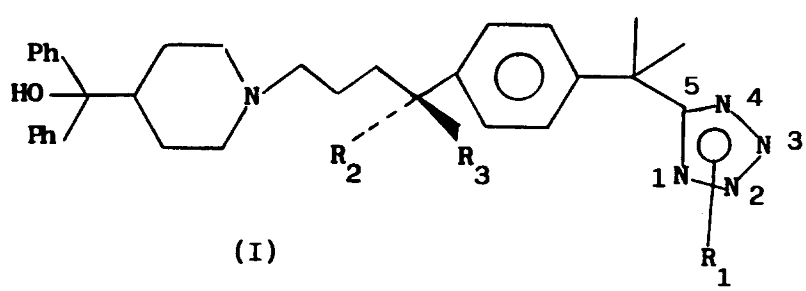

(I)

where $R_1$, which is in position 1 or 2 of the tetrazole ring, stands for H, or a linear or branched alkyl group containing from 1 to 6 carbon atoms; $R_2$ and $R_3$, different from one another, are selected between H and OH, and their pharmaceutically acceptable salts.

EP 0 648 759 A1

## Field of the invention

The present invention relates to terfenadine derivatives having antihistaminic activity, the process for their preparation and the relative pharmaceutical compositions.

## Prior art

Terfenadine, i.e. α-[4-(1,1-dimethylethyl)phenyl]-4-(4-hydroxyphenylmethyl)-1-piperidinebutanol, an antihistaminic compound commercially available as a variety of products (Seldan®, Teldane®), is a selective antagonist of histaminergic receptor $H_1$ and is used in the theraphy for the treatment of seasonal allergic rhinitides and of other diseases for which histamine is held to be responsible.

Terfenadine is considered a choice antiallergic, compared with the traditional antihistamines, such as chlorpheniramine, diphenhydramine, promethazine, and triprolidine, as it produces on the central nervous system less important untoward side effects of the anticholinergic, antiserotoninergic, antiadrenergic, and antihistaminic type (psychomotor disorders, sedation, dryness of fauces (J.K. Woodward, N.L. Munro, Arzneim. Forsch. Drug Res., **32**, 1154, 1982; A.N. Nicholson, Arzneim. Forsch. Drug Res., **32**, 1191, 1992). This notwithstanding, in patients suffering from liver complaint or being administered drugs slowing down the metabolism (Ketoconazole, erythromycin), terfenadine may produce effects on the heart rhythm that may result even fatal (Chem. Eng. News, p. 26, June 1993).

It may be hypothesized that the observed cardiotoxic effects are due to the fact that the metabolism of terfenadine is slowed down by the administration of the above drugs.

Terfenadine biotransformation in man produces a series of metabolites, the principal being 4-[1-hydroxy-4-(4-hydroxydiphenylmethyl)piperidinyl]butyl-α,α-dimethylphenylacetic acid and azacyclonol.

The carboxylated metabolite exhibits a potent antihistaminic activity and one of its two enantiomers has been assumed to be responsible for cardiotoxic phenomena.

In the organism, the carboxylated metabolite undergoes a variety of metabolic reactions (such as conjugation reactions) favouring the elimination of same before an optimal therapeutic activity is achieved.

## Detailed description of the invention

The present invention relates to terfenadine derivatives of formula (I)

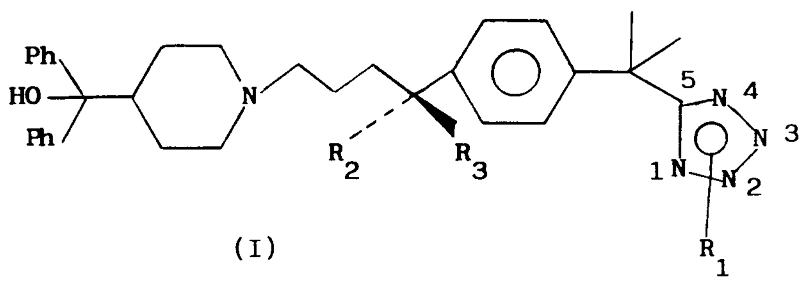

(I)

where $R_1$, which is in position 1 or 2 of the tetrazole ring, stands for H, or a linear or branched alkyl group containing from 1 to 6 carbon atoms; $R_2$ and $R_3$, different from one another, are selected between H and OH, and the pharmaceutically acceptable salts thereof.

When $R_1$ = H, structures $(I)_1$ and $(I)_2$, where $R_1$ = H is in position 1 and 2, respectively, describe the two tautomeric forms of the same compound.

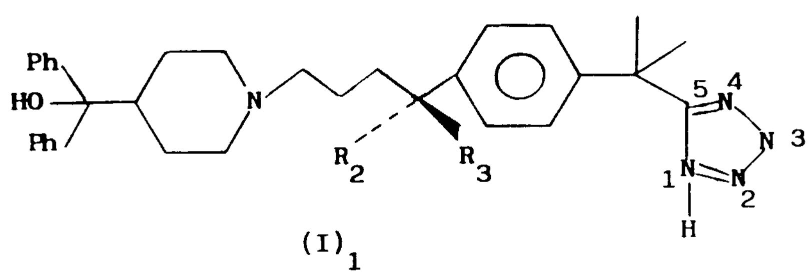

$(I)_1$

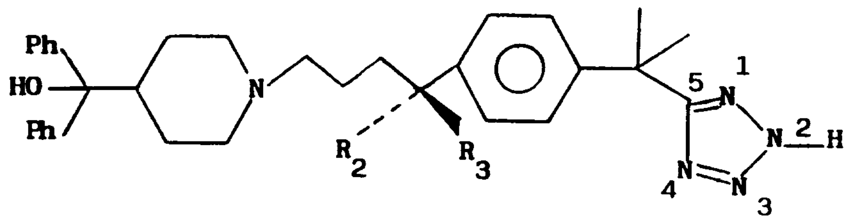

$(I)_2$

As used herein, the term "pharmaceutically acceptable salts" refers to either inorganic or organic salts with acids or with bases.

The salification of derivatives of formula (I) is carried out according to conventional techniques.

$R_1$ may be, for instance, methyl or ethyl.

The derivatives of formula (I) contain an asymmetric carbon atom, i.e. a carbon atom substituted with a hydroxyl group ($R_2$ or $R_3$) in position 4 of the butyl chain, and may exist in an optically active form or as racemic mixtures.

In the derivatives of formula (I) where $R_2$ = H and $R_3$ = OH, the configuration of the asymmetric carbon atom is S, whereas in the derivatives where $R_2$ = OH and $R_3$ = H said configuration is R.

It has surprisingly been found that the derivatives of formula (I) and the compositions containing same exhibit a better antihistaminic activity than terfenadine.

Salient and unexpected advantages of the present terfenadine derivatives are that they

a) show a high antihistaminic activity;

b) have a remarkable biological stability and secure an effective and long-lasting therapeutic activity, i.e. they produce a more potent effect and of longer duration than the carboxylated metabolite of terfenadine;

c) do not give rise to any toxicity problem connected with changes in hepatic metabolism;

d) do not show any anticholinergic activity at the dosages securing an antihistaminic activity; therefore, they produce reduced side effects on the central nervous system, such as sedation, than other antihistaminic molecules;

e) exhibit an antihistaminic activity of essentially peripheral type.

In particular, when compared with terfenadine, said derivatives produce less important side effects on the central nervous system, such as sedation.

In the case of the derivative of formula (I) where $R_1$ = H, the lower incidence of the side effects on the central nervous system may be attributed to the greater difficulty with which said compound crosses the blood-brain barrier.

The derivatives of formula (I) are useful in human therapy, in particular for the treatment of various pathological states for which histamine is held to be responsible, such as allergic diseases, with particular reference to seasonal allergic rhinitides and asthma, as well as ulcerative conditions of the gastrointestinal apparatus.

Among the derivatives of the present invention, particularly preferred is p-[1-hydroxy-4-(4-hydroxydiphenylmethyl-1-piperidinyl)butyl]-2-(1H-tetrazol-5-yl)-isopropylbenzene (derivative of formula (I) where $R_1$ = H), in the form both of single enantiomers (enantiomer S, where $R_2$ = H and $R_3$ = OH, and enantiomer R, where $R_2$ = OH and $R_3$ = H) and of mixtures thereof, such as the racemic mixture.

It is a further object of the present invention to provide a process for the preparation of said derivatives both as racemic mixtures and in the form of single enantiomers, comprising the following steps:

a) reducing $\alpha,\alpha$-dimethylphenylacetic acid to give 2-methyl-2-phenylpropanol;

b) protecting the alcohol group of 2-methyl-2-phenylpropanol to give the compound of formula (II)

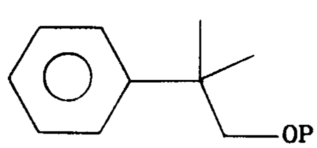

(II)

where P is a protective group of the alcoholic moiety which is stable under the successive acylation conditions;

c) acylating the aromatic ring of the compound of formula (II) by reacting it with the acyl halide of formula (III)

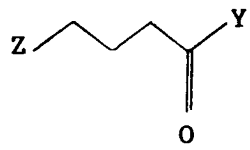

(III)

where Y and Z, identical or different, are halogens, to give the ketone of formula (IV)

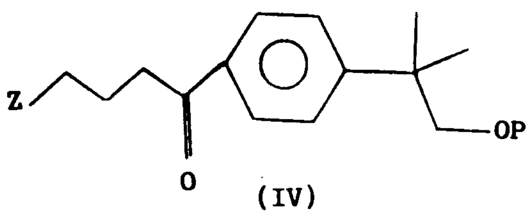

(IV)

where P and Z are as defined above;

d) protecting the ketone moiety of the aforesaid compound of formula (IV) by reacting it with a hydroxylated compound, to give the ketal of formula (V)

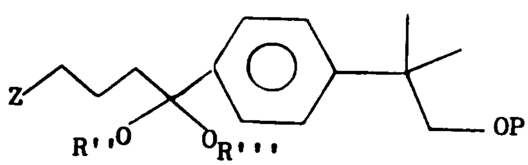

(V)

where P and Z are as defined above, R'' and R''', identical or different, are each linear or branched alkyl groups containing from 1 to 6 carbon atoms, or, taken together, stand for a linear or branched aliphatic residue which, together with the oxygen atoms to which it is bound, forms a heterocyclic ring of 5 to 7 atoms;

e) deprotecting the alcohol moiety of the product obtained according to step d) to give the product of formula (VI)

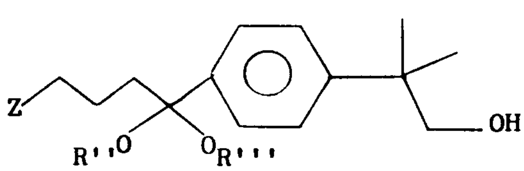

(VI)

where Z, R'' and R''' are as defined above;

f) oxidizing the alcohol moiety of the aforesaid product of formula (VI) to give the carboxylic acid of formula (VII)

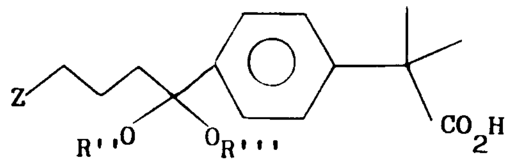

(VII)

where Z, R'' and R''' are as defined above;

g) converting of the carboxylic acid of formula (VII) into the acyl halide of formula (VIII)

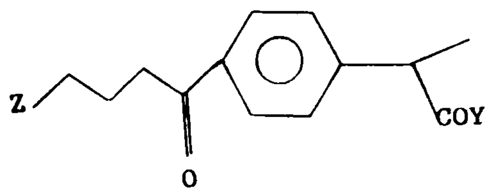

(VIII)

where Z and Y are as defined above;

h) treating the compound of formula (VIII) with ammonia to give the corresponding amide of formula (IX)

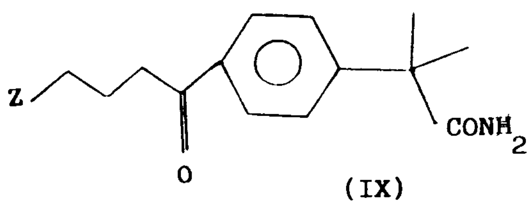

(IX)

where Z is as defined above;

i) converting the amide of formula (IX) into the nitrile of formula (X)

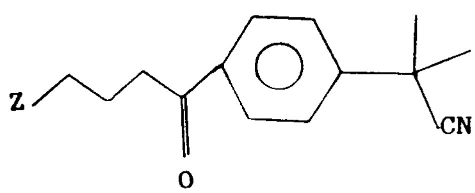

(X)

where Z is as defined above;

l) reducing the ketone moiety of the compound of formula (X) to give the alcohol derivative of formula (XI)

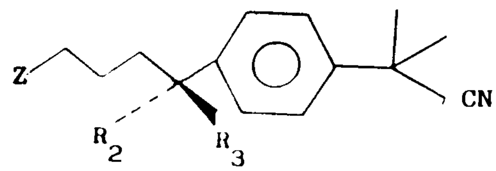

(XI)

where $R_2$, $R_3$ and Z are as defined above;

m) condensing the derivative of formula (XI) with azacyclonol to give the compound of formula (XII)

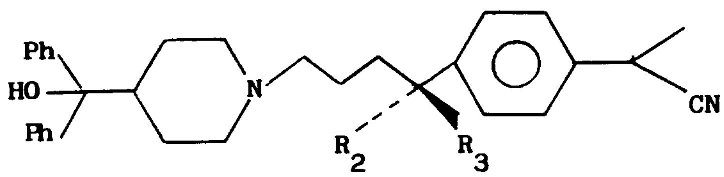

(XII)

where $R_2$ and $R_3$ are as defined above;

n) forming the tetrazole ring by reacting the cyano-derivative of formula (XII) with an azide to give the derivative of formula (I), where $R_1$ is H and $R_2$, $R_3$ are as defined above;

o) optionally, alkylating the tetrazole ring with an alkylating agent able to transfer linear or branched alkyl groups containing from 1 to 6 carbon atoms, to give the derivative of formula (I), where $R_1$ is a linear of branched alkyl containing from 1 to 6 carbon atoms and $R_2$ and $R_3$ are as defined above.

α,α-Dimethylphenylacetic acid is a known compound and its preparation is described in literature (C. Giordano et al., Synthesis, 436, 1985).

The Applicant has now found that when α,α-dimethylphenylacetic acid is prepared by rearrangement of 2-bromoisobutyrophenone with $ZnBr_2$, in excess in respect of the starting ketone (in particular 8 to 15 $ZnBr_2$ moles per starting ketone mole), using in methanol as a reaction solvent, at +100 °C - +130 °C, then carrying out hydrolysis at 70 °C to 90 °C of the product obtained by water addition, followed by extraction with an organic solvent (e.g. alkaline hydrolysis by treatment with excess NaOH in methanol/water), 98% pure $ZnBr_2$ maintained under high vacuum for at least 12 hours is conveniently used. The α,α-dimethylphenylacetic acid recovered by acidification and extraction with organic solvents is purified by extracting the organic phases thus obtained with an alkaline aqueous solution, e.g. a saturated $NaHCO_3$ solution, acidifying the alkaline aqueous phase with a strong mineral acid, such as HCl, and separating the precipitated α,α-dimethylphenylacetic acid, e.g. by filtration.

2-Bromoisobutyrophenone can be prepared as described in Beilstein, 7, 316.

The reduction as per step a) is carried out with a reducing agent of the carboxylic moiety in an anhydrous aprotic solvent at a temperature preferably of from 0 °C to +40 °C.

Among the several reducing agents of the carboxylic moiety that may be used, $LiAlH_4$ is particularly preferred.

The solvents conveniently used are ethereal solvents (tetrahydrofuran, ethyl ether).

According to preferred conditions, the reducing agent is $LiAlH_4$, the anhydrous aprotic solvent is ethyl ether, the reaction temperature is comprised between +20 °C and +30 °C.

$LiAlH_4$ is preferably used in excess in respect of the substrate, generally from 5 to 10 moles per mole of compound to be reduced, said excess being destroyed at the reaction end by adding methanol, at the aforesaid reaction temperature. The resulting product is recovered after treatment with a strong acid, such as sulphuric acid, up to complete solubilization of the solid phase present in the reaction mixture.

In step b) the alcoholic moiety is protected so to obtain derivatives which are stable under the successive acylation conditions.

Protective group P is preferably an acyl group, such as for example an acetyl group.

The protection of 2-methyl-2-phenylpropanol is carried out by treatment with an acylating agent, such as for instance acyl halides or anhydrides, optionally in the presence of an organic solvent, in the presence of an organic base, at a temperature of from 0 °C to 50 °C.

The organic solvents used are those inert under the acylation conditions, such as for example halogenated solvents (chloroform, methylene chloride).

The organic bases are preferably tertiary alkylamines containing from 3 to 20 carbon atoms, such as triethylamine, aromatic heterocyclic bases, such as pyridine, dimethylaminopyridine and 4-pyrrolydinepyridine, either alone or as mixtures thereof.

In a more preferred embodiment of the present invention, P is the acetyl group, the acylating agent is acetic anhydride, the organic base is a mixture of triethylamine and 4-pyrrolidinepyridine, the organic solvent is methylene chloride, the reaction temperature is +15 °C to +30 °C.

Typically, acetic anhydride is used in a 3 to 5 times as moles excess in respect of the substrate to be acylated, and the organic base is also in a 3 to 5 times as moles excess.

Step c) is a Friedel-Craft acylation carried out in the presence of a Lewis acid, in inert organic solvents, at a temperature of from -20 °C to +20 °C.

Among the Lewis acids that may be used, $AlCl_3$ is particularly preferred.

The organic solvents that may be used are aromatic solvents deactivated towards aromatic eletrophilic substitutions, such as nitrobenzene, halogenated solvents or sulphides, such as $CS_2$.

In a preferred embodiment of the present invention, the product of formula (II) where P is acetyl, and the acyl halide of formula (III) where Y=Z=Cl, are used, in the presence of $AlCl_3$ as a Lewis acid and of $CS_2$ as reaction solvent, at a temperature of 0 °C to 5 °C.

In a more preferred embodiment of the present invention, acylation is carried out by addition of the derivative of formula (III) to an $AlCl_3$ suspension, in excess in respect of the acyl halide, typically of from 1.2 to 2 moles per mole, followed by addition of the substrate of formula (II), and allowing the reaction to proceed at the temperature defined above.

Friedel-Craft acylation unexpectedly proved to be more selective in the formation of the para isomer if carried out on the derivative of formula (II) rather than on other compounds, such as the α,α-dimethylphenylacetic acid esters. In fact, comparative acetylation tests carried out on methyl ester of α,α-dimethylphenylacetic acid and on 1-acetoxy-2-methyl-2-phenylpropanol with 4-chlorobutyryl chloride as the acylating agent, in $CS_2$, in the presence of $AlCl_3$, showed in the former case the formation of equimolecular amounts of the para and meta isomers of the corresponding acylation product, whose separation is extremely lengthy and expensive, and in the latter case the formation of no more than 10% of the meta isomer.

Furthermore, Friedel-Craft acylation of 2-phenyl-2-methylbutyronitrile with 4-chlorobutyryl chloride, in $CS_2$, in the presence of $AlCl_3$, carried out under conditions analogous to those adopted in the presents process, gives the corresponding derivative acylated in para position in lower yields in comparison with those obtained by the process described herein via steps a) through i).

The ketalization as per step d) is necessary to avoid cyclisation of the compound of formula (IV) during the successive step of deprotection of the hydroxyl moiety:

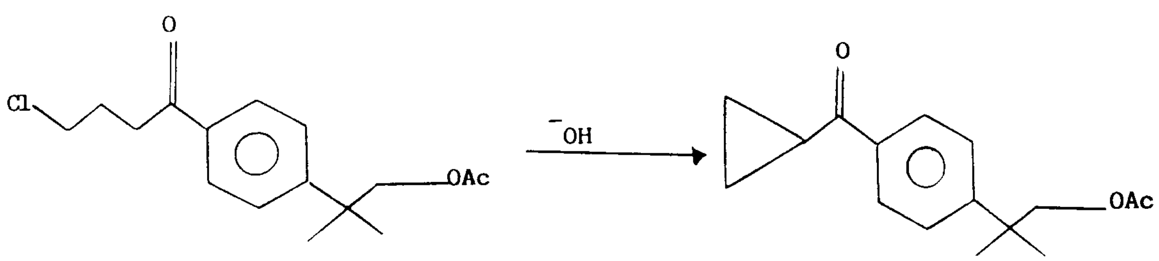


In step d) the hydroxylated compound is preferably a diol, whose aliphatic residue, either linear or branched, contains from 2 to 12 carbon atoms, such as ethylene glycol, and the reaction is carried out in an anhydrous aprotic solvent, in the presence of an acid catalyst, at a temperature of from 50 °C to 100 °C.

The separation of water may be carried out under different conditions, by azeotropic distillation or by appropriate dehydrating agents. Preferably, water is removed by azeotropic distillation, in which case aromatic solvents, such as benzene or toluene, are conveniently used.

The acid catalyst is a strong acid, such as p-toluenesulphonic acid, trifluoroacetic acid, gaseous HCl.

Under preferred conditions the compound of formula (IV) has Z=Cl and P=acetyl, the hydroxylated compound is ethylene glycol, the anhydrous aprotic solvent is benzene, the acid catalyst is p-toluenesulphonic acid, the reaction is carried out at the reflux temperature of the reaction mixture, and the water that forms is distilled azeotropically at atmospheric pressure. The resulting product is the ketal of formula (V),

wherein R'' and R''', taken together, stand for a $-(CH_2)_2-$ residue, and Z is Cl.

The deprotection as per step e) is preferably carried out by treating the derivative of formula (V) with a strong inorganic base, e.g. an alkaline hydroxide, in an alcoholic solvent, at a temperature of 0 ° C to 50 ° C.

According to further preferred conditions, the derivative of formula (V), where Z is Cl, P is acetyl and R'' and R''', taken together, stand for $-(CH_2)_2$, is deprotected using a stoichiometric excess of NaOH, in methanol, at a temperature of from 15 ° C to 30 ° C.

The oxidation of the alcoholic moiety to an acid is preferably carried out with Jones's reagent, i.e. a solution of chromic acid in a mixture of sulphuric acid in water, by adding the reagent under controlled conditions, so to maintain the pH of the reaction mixture neutral, at a temperature of -20 ° C to +20 ° C, in an organic solvent which is inert under the oxidation conditions, such as acetone.

With a neutral pH, ketal hydrolysis is minimized and high yields of the desired product are obtained.

According to further preferred conditions, the substrate to be oxidized is the derivative of formula (VI), wherein Z is Cl and R'' and R''', taken together, stand for $-(CH_2)_2$; Jones's reagent is used, in acetone, titrating the substrate to be oxidized until the reaction mixture turns the same colour as Jones's reagent, at a temperature of from 0 ° C to +5 ° C.

Once the solid phase present in the reaction mixture has been separated, the acid obtained is extracted with an organic solvent, then recovered after washing with water, drying and evaporating the organic phase.

Step g) is the conversion of the carboxylic moiety into an acyl halide with simultaneous deprotection of the ketalic moiety.

Step g) may be conveniently carried out using a halogenating agent, such as thionyl chloride or thionyl bromide, in excess in respect of moles of starting compound, and operating at a temperature of from 0 ° C to 40 ° C, in an anhydrous medium, optionally in the presence of an organic aprotic solvent.

According to preferred conditions, the substrate used is the compound of formula (VII) wherein Z = Cl and R'' and R''', taken together, stand for $-(CH_2)_2$, the halogenating agent is thionyl chloride, used as a reaction solvent, in the absence of organic solvents, and in an excess of 1.5 to 2 times as moles in respect of the substrate to be halogenated; the reaction temperature is 15 ° C to 30 ° C.

Step h) is preferably carried out at a temperature of -20 ° C to +20 ° C, in an aqueous medium.

According to more preferred conditions, step h) is carried out on the derivative of formula (VIII), where Y = Z = Cl, by addition of a solution of the product of formula (VIII) in an anhydrous ether solvent, such as tetrahydrofuran or ethyl ether, to aqueous ammonia, in stoichiometric excess in respect of the product of formula (VIII), at 0 ° C.

In step i) the amide of formula (IX) is treated with a dehydrating agent, in an anhydrous aprotic solvent, in the presence of a base, at a temperature of 40 ° C to 100 ° C; then, the raw product recovered from the reaction mixture is treated with a strong acid, in an aprotic solvent, at a temperature of from 0 ° C to 40 ° C.

The treatment with a strong acid is preferably carried out on the raw product recovered from the reaction mixture after filtration of the insoluble residue and extraction with an organic solvent.

According to further preferred conditions, the compound of formula (IX) is the one having Z = Cl, the dehydrating agent is $POCl_3$, the base is $Na_2CO_3$, the anhydrous aprotic solvent is acetonitrile and the temperature of treatment with the dehydrating agent is the reflux temperature of the reaction mixture; in the successive treatment with acids, the acid is concentrated hydrochloric acid, the aprotic solvent is tetrahydrofuran, the temperature is of from 15 ° C to 30 ° C.

Typically, when compared with the compound of formula (IX), the dehydrating agent content ranges from 1.2 to 1.8 moles per mole, the base content in moles ranges from 0.3 to 0.8 times and the acid is used in excess from 5 to 10 moles per mole.

The reduction of the ketone moiety to an alcohol according to step l) may be carried out with an optically inactive reducing agent, such as sodium borohydride, yielding the derivative of formula (XI) in racemic form, which is then converted, by steps m) through o), into the corresponding derivative of formula (I), which is also in racemic form.

Instead, when step l) is carried out with an optically active reducing agent, such as B-chlorodiisopinocampheyl borane, the corresponding derivatives of formula (XI) in optically active form are obtained; in particular, enantiomer S is obtained when using the levorotatory form (-) of said borane and enantiomer R when using the dextrorotatory form (+) of said borane.

The optically active isomers of B-chlorodiisopinocampheyl borane are both commercially available.

The successive steps of the synthesis are carried out under conditions analogous to those described for the synthesis of derivative of formula (I) in racemic form, with conversion of forms S and R of the derivative of formula (XI), respectively, into forms S and R of derivatives of formula (XII) and of derivatives of formula (I), as per steps m) through o) of the present process.

The reduction of the ketone moiety with sodium borohydride is typically carried out in the presence of an anhydrous organic solvent selected among alcoholic solvents, ether solvents, and mixtures thereof, at a temperature of -20 °C to +20 °C.

Under preferred conditions, the compound of formula (X) has Z = Cl, the solvent is a mixture of methanol and tetrahydrofuran, the temperature is 0 °C to 5 °C.

Typically, sodium borohydride is used in an excess of from 3 to 8 moles per substrate mole in respect of the substrate to be reduced.

The reduction with optically active B-chlorodiisopinocampheyl borane is typically carried out in an anhydrous organic solvent (e.g. an ether solvent, such as ethyl ether or tetrahydrofuran), at a temperature of - 40 °C to 0 °C; the resulting intermediate borate is then hydrolyzed by treatment with a hydroxylated compound at a temperature generally ranging from 20 °C to 30 °C.

The treatment is preferably carried out on the nitrile of formula (X), where Z = Cl, in anhydrous tetrahydrofuran, at -25 °C for a period of about 2 hours, and the reaction is then maintained at room temperature (about 25 °C) for a further period of about 24 to 48 hours; the resulting borate intermediate is hydrolyzed by treatment with diethanolamine, in anhydrous ethyl ether, at room temperature (about 25 °C) to give the corresponding derivative of formula (XI).

Typically, B-chlorodiisopinocampheyl borane is used in excess in respect of the substrate of formula (X), in particular in an amount of from 1.1 to 1.8 moles per substrate mole.

The treatment with diethanolamine is conveniently carried out with 3 to 3.5 moles of diethanolamine per mole of substrate of formula (X).

The condensation with azacyclonol is preferably carried out in the presence of an organic or inorganic base, such as an alkaline or alkaline earth carbonate or bicarbonate, in the presence of a catalytic amount of an alkaline iodide, in an anhydrous medium, in a slightly polar aprotic solvent (e.g. an aromatic solvent, such as toluene), or in a strongly polar solvent (e.g. dimethylformamide), either individually or as a mixture thereof, at a temperature of 80 °C to 150 °C.

According to further preferred conditions, the derivative of formula (XI) has Z = Cl, the alkaline iodide is KI, the base is anhydrous potassium carbonate, the solvent is a mixture of toluene and dimethylformamide, the temperature is the reflux temperature of the reaction mixture, and the reaction water is separated as it forms.

Compared with the derivative of formula (XI), the alkaline iodide content ranges from 5 to 15% by mol and the base is at least in a stoichiometric amount, typically of from 2 to 5 moles per mole.

The resulting nitrile may be conveniently purified by chromatography on silica gel, eluting with chloroform and with chloroform-methanol mixtures.

The formation of the tetrazole ring is obtained by treating the derivative of formula (XII) with an azide, e.g. trimethylsilylazide, tributyltinazide and sodium azide, in an anhydrous medium, using as organic aprotic solvents, ether solvents, aromatic solvents, and mixtures thereof, at a temperature of 15 °C to 180 °C.

Depending on the reagent used, further chemical treatments may be required to convert the intermediates formed by reacting the nitrile moiety with azide into the desired tetrazole derivatives. Said treatments are selected by those skilled in the art on the basis of the information available for the synthesis of tetrazole rings. For example, as concerns trimethylsilylazide, the product recovered from the reaction mixture has to be treated to remove the silyl group, e.g. with aqueous alcohols.

In particular, according to the present process, the following conditions may be adopted:

- trimethylsilylazide in tetrahydrofuran, in autoclave, at 100 °C to 140 °C;
- trimethylsilylazide, in the presence of diethylazodicarboxylate and $Ph_3P$, in tetrahydrofuran, at room temperature;
- tributyltinazide in toluene, at reflux temperature;
- sodium azide, in the presence of ammoniun chloride, in dimethylformamide, at 110 °C to 120 °C:
- sodium azide, in the presence of $Et_3N.HCl$ and of 1-methyl-2-pyrrolidinone, at 150 °C.

The product derived from the reaction with azide is purified by chromatography on silica, eluting, e.g. with $CHCl_3$/MeOH/hexane mixtures. According to more preferred conditions, trimethylsilylazide is used, the solvent is tetrahydrofuran, the temperature is 100 °C to 140 °C, and the product obtained after solvent evaporation is treated with methanol/$H_2O$ at reflux temperature.

The raw product recovered from the reaction mixture is purified by chromatography on silica eluting with $CHCl_3$/MeOH/hexane mixtures.

Alternatively, the intermediate formed by reacting the compound of formula (XII) with trimethylsilylazide is treated with a quaternary ammonium fluoride, e.g. tetrabutylammonium fluoride, in an anhydrous ether solvent, such as tetrahydrofuran, typically at a temperature of from 20 °C to 60 °C.

The treatment with $BU_4NF$ is typically carried out when step n) is carried out on the derivative of formula (XII) in optically active form [obtained from the corresponding optically active derivatives of formula (XI)].

Typically, the azide is used in excess in respect of the substrate, e.g. from 5 to 15 times as moles.

The derivatives of formula (I), where $R_1$ is different from hydrogen, are preferably prepared by treating the derivative of formula (I), where $R_1$ is H, with an alkylating agent $R_1W$, where $R_1$ is a linear or branched alkyl group containing from 1 to 6 carbon atoms, and W is a halogen, preferably Br, in the presence of a base, in an anhydrous solvent. For instance, it is possible to use an alkaline alkoxide in an alcoholic solvent, or an alkaline hydride in an alcoholic solvent or in an ether solvent (ethyl ether, tetrahydrofuran). According to typical conditions, $MeO^{-}Na^{+}$ in methanol is used.

It is a further object of the present invention to provide pharmaceutical compositions containing as active ingredient an effective dose of at least one derivative of formula (I) or of a pharmaceutically acceptable salt thereof, in combination with suitable excipients.

Said compositions are particularly useful in human therapy for the treatment of the diseases mentioned above.

To attain the desired therapeutic effects, the product may be administered by different routes, by the oral and parenteral routes, in the pure form or in the form of pharmaceutical compositions.

The formulations of appropriate pharmaceutical compositions may be produced according to traditional techniques, as described e.g. in Remington's Pharmaceutical Sciences Handbook, Hack Publishing Co., USA, 16th ed. (1980).

The compositions of the invention may be administered, on average, once or twice a day; however, more frequent administrations may be convenient, at least in some cases, their number depending on the patient's conditions and on the way of administration chosen. For oral administration, the composition may be provided in the solid or liquid form, such as capsules, pills, tablets, powder, solution, suspension or emulsion, and optionally delayed release forms.

A solid dosage unit may be a gelatin capsule, either soft or hard, containing inert lubricants and excipients, such as lactose, saccharose or starch.

The compounds of the invention may also be formulated as tablets using conventional excipients, such as lactose, saccharose, starch, gelatin, alginic acid, stearic acid, magnesium stearate, etc.

For parenteral administration, the compounds are administered as injectable formulations, either dissolved or suspended in pharmacologically acceptable diluents, a sterile carrier such as water or an oil, with or without addition of other compounds.

The oils that may be used are vegetable or animal or mineral or synthetic oils, such as peanut oil, soybean oil and mineral oil.

The carriers generally used for the solutions for injection are water, aqueous solutions of mineral salts, and aqueous solutions of dextrose or of other sugars, ethanol, glycols, such as propylene glycol or polyethylene glycol.

The compositions being the object of the present invention may contain, in addition to the aforesaid excipients and active ingredient, also other active ingredients exerting a complementary or in any case useful activity.

The following examples are conveyed by way of illustration, not of limitation of the present invention.

**Brief Description of the Drawings**

**Fig. 1** is a diagram reporting the percentage of $H_1$ receptors from cerebral cortex occupied by increasing doses of ligands TER, MET I and TFZ by displacement of [$^3$H]mepyramine.

**Fig. 2** is a diagram representing the percentage of $H_1$ receptors from guinea pig ileum occupied by increasing doses of ligands TER, MET I and TFZ by displacement of [$^3$H]mepyramine.

**Fig. 3** is a Scatchard's diagram relating to the saturation bond for [$^3$H]mepyramine in the absence (□) or in the presence (■) of TFZ (2 x $10^{-7}$ M), reporting the ratio of bound (B) (nM) to free (F) (pmoles) $^3$H mepyramine versus the ratio of bound (pmoles) of $^3$H mepyramine per mg of protein and showing the value of $B_{max}$ (maximal binding sites of receptor systems as pmoles of $^3$H mepyramine per mg of protein).

**Fig. 4** is a graphical representation according to Lineweaver-Burk of the antihistaminic activity of TFZ, assayed on guinea pig isolated ileum in the absence of antagonist (A) and in the presence of three different TFZ concentrations (B, C and D), using hystamine concentrations [$10^{-8}$ M] of 6.8 (A), 3.4 (B), 1.7 (C) and 0.85 (D), respectively. The contraction effect (E) detected is plotted against the reciprocal of Histamine concentration [Hist].

**Fig.5** represents the inhibition of histamine-submaximal contraction (hystamine $6.8x10^{-8}$ M) exerted by MET I (A) or TFZ (B).

**Fig. 6** reports the ACh agonist activity on guinea pig isolated ileum in the absence and in the presence of TFZ (5 x 10 $^{-7}$ M).

## EXAMPLE 1

### Preparation of α,α-dimethylphenylacetic acid

A three-necked flask, adequately flamed and maintained in an inert environment was fed with 300 g of $ZnBr_2$ (1.3 moles; 98% pure; maintained under high vacuum at 100 ° C for at least 12 hours) and with 130 ml of MeOH (distilled twice). The addition was highly exothermic.

Zinc bromide was allowed to dissolve completely, the temperature was raised to 115 ° C, then 2-bromoisobutyrophenone (19 ml; 0.113 mol) was slowly added. Once the addition was completed, the mixture was left under stirring in an inert environment at 115 ° C for 5 hours. The mixture was added with 300 ml of $H_2O$ and ice and extracted with $CH_2Cl_2$ (3 x 150 ml).

The combined organic phases, after drying over sodium sulphate, were filtered, and evaporated to dryness. The raw product obtained was dissolved in a refluxing mixture of $H_2O$ (70 ml) and MeOH (170 ml) in an inert environment. The mixture was allowed to cool for approx. 5 min and added with 4 g of NaOH (0.1 moles). The temperature was raised to 80 ° C and the reaction was continued in an inert environment for 12 hours. $H_2O$ (200 ml) and ice were added. The mixture was acidified with 10% HCl and extracted with $CH_2Cl_2$ (3 x 200 ml). The combined organic phases were extracted with an $NaHCO_3$ saturated solution (4 x 200 ml), then the combined aqueous phases were acidified with 10% HCl.

A flaky white solid was obtained, which was filtered and evaporated to dryness. 13 g of α,α-dimethylphenylacetic acid was obtained. Yield 65% (m.p. 80 ° C).

$^1$H-NMR ($CDCl_3$) δ: 7.45-7.2 (m,5H,Ar-H); 1.6 (s,6H,$ArC(CH_3)_2COOH$.

$^{13}$C-NMR ($CDCl_3$) δ: 183.348(-COOH); 143.765 (C quat.Ar); 128.379; 126.879; 125.735 (Ar); 46.245 (Ar-$C(CH_3)_2$-); 26.145 (Ar-$C(CH_3)_2$-).

## EXAMPLE 2

### Preparation of the derivative of formula (I), where $R_1$ is H [racemic mixture of the enantiomer where $R_2$=H, $R_3$=OH and of the enantiomer where $R_2$=OH and $R_3$=H]

a) <u>2-Methyl-2-phenylpropanol</u>

A solution of $LiAlH_4$ (15 g; 394 mmoles) in anhydrous ethyl ether (200 ml), kept under stirring in an inert environment at 25 ° C, was slowly added with 10 g of substrate from the previous step (61 mmoles), solubilized in 400 ml of anhydrous $Et_2O$. Once the reaction was completed, excess $LiAlH_4$ was destroyed by slow addition of a 3:15 mixture of MeOH/$Et_2O$ (570 ml). The solid was solubilized with 1400 ml of 5% $H_2SO_4$. A clear solution was obtained and extracted with $Et_2O$ (4 x 400 ml). The combined organic phases were washed with a 5% $NaHCO_3$ solution (1 x 500 ml) and treated with brine (1 x 500 ml); then, after drying over sodium sulphate, were filtered and evaporated to dryness. 8.9 g of product was obtained. Yield 91.8%.

$^1$H-NMR ($CDCl_3$) δ: 7.5-7.1 (m,5H,Ar); 3.6(s,2H,(-$CH_2$-OH); 1.5 (brs, O-H); 1.3 (s,6H,-$C(CH_3)_2$-).

b) <u>1-Acetoxy-2-methyl-2-phenylpropanol</u>

A solution of the substrate from the previous step (16 g; 107 mmoles) in $CH_2Cl_2$ (600 ml) was added with 30 ml of acetic anhydride (3181 mmoles), 45 ml of triethylamine (345 mmoles) and 300 mg of 4-pyrrolidinepyridine.

The mixture was left under stirring at room temperature for approx. 20 min, then the solvent was concentrated. The residue was taken up with ethyl ether (500 ml); then the product was washed with $H_2O$ (4 x 300 ml), treated with brine (1 x 300 ml). The organic phase was dried over sodium sulphate, filtered and the solvent evaporated. 19.9 g of product were obtained. Yield 96%.

$^1$H-NMR ($CDCl_3$) δ: 7.4-7.1 (m,5H,Ar); 4.1 (s,2H,-$CH_2OAc$; (s,3H,-$OCOCH_3$); 1.4 (s,6H,Ar-$C(CH_3)_2$-).

c) 1-Acetoxy-2-methyl-2-[4-(4-chlorobutyryl)-]phenylpropanol

A suspension of $AlCl_3$ (14 g, 104 mmoles) in $CS_2$ (14 ml) was added with 7.6 ml of 4-chlorobutyrylchloride (67.7 mmoles) and kept under stirring for approx. 15 min. after bringing the temperature to 0 °C. 10 g of substrate from the previous step (52 mmoles), dissolved in 50 ml of $CS_2$, were added over a period of approx. 20 min.. Stirring was continued at 0 °C for approx. 30 min., then 7 ml of $CS_2$ and 7 g of $AlCl_3$ (52.5 mmoles) were added. A temperature of 0 °C to 5 °C was maintained for approx. 1 hour. Once the reaction was completed, the mixture was poured into a beaker containing acidulated water (400 ml) and ice, and the reaction flask was washed with $CH_2Cl_2$. The mixture was left under stirring for about 2 hours, then the organic phase was separated, and the aqueous phase was extracted with $CH_2Cl_2$ (3 x 200 ml). The combined organic phases were treated with an $NaHCO_3$ saturated solution (2 x 200 ml) and with brine (1 x 200 ml); after drying over $Na_2SO_4$, they were filtered and evaporated to dryness. 14 g of raw product were obtained, which were purified by flash chromatography eluting with $Et_2O$/petroleum ether, 2:8.

9.8 g of product containing approx. 9% of the isomer isomer were obtained. Total yield 60%.

$^1$H-NMR ($CDCl_3$) δ: 7.95 (d,2H,J = 8.5Hz,Ar); 7.45(d,2H,J = 8.5Hz, Ar); 4.2 (s,2H,-$CH_2$-OAc); 3.7 (t,2H,Cl-$CH_2$-); 3.2 (t,2H,-$CH_2$-CO-Ar); 2.2 (m,2H,Cl-$CH_2$-$CH_2$-); 2 (s,3H,$CH_3$COO); 1.4 (6H, -C($CH_3$)$_2$-).

d) 1-Acetoxy-2-methyl-2-[4-(1-ethylenedioxy-4-chlorobutyl)]-phenylpropanol

A solution of product from the previous step (18 g, 60 mmoles) in anhydrous benzene (700 ml), maintained in an inert environment, was added with 5 ml of ethylene glycol (89 mmoles) and 400 mg of p-toluenesulphonic acid. The reaction mixture was refluxed, kept under stirring in a nitrogen environment for 24 hours, and the reaction water was separated.

The solvent was evaporated; the residue was taken up with EtOAc (350 ml).

The product was washed with $H_2O$ (4 x 200 ml). The organic phase was treated with an NaCl saturated solution (1 x 200 ml) and, after water elimination and filtration, the solvent was evaporated to dryness. The resulting product was purified by flash chromatography eluting with petroleum ether/$Et_2O$, 8:2.

15 g of product containing approx. 15% of the starting product was obtained. Total yield 70%.

$^1$H-NMR ($CDCl_3$) δ: 7.35 (m,4H,Ar); 4.1 (s,2H,(-$CH_2$-OAc; 4-3.7 (2 symmetric m, 4H, ketal); 3.5 (m,2H,-$CH_2$-Cl); 2 (s,3H,$CH_3$-COO-); 2 (m,2H,-$CH_2$-C (ketal)-); 1.85 (m,2H,Cl-$CH_2$-$CH_2$); 1.4 (s,6H,-C($CH_3$)$_2$).

e) 2-Methyl-2-[4-(1-ethylenedioxy-4-chlorobutyl)]-phenylpropanol

15 g of substrate from the previous step (40 mmoles) were treated with 300 ml of 2% NaOH in MeOH.

The mixture was stirred at room temperature for 30 min., added with $H_2O$ (200 ml) and extracted with EtOAc (3 x 300 ml). The combined organic phases were treated with an NaCl solution and, after drying over $Na_2SO_4$, were filtered and evaporated to dryness. 14.4 g of product were obtained (quantitative yield).

$^1$H-NMR ($CDCl_3$) δ: 7.35 (m,4H,Ar); 4-3.7 (2 symmetric m, 4H, ketal); 3.6 (s,2H,-$CH_2$-OH); 3.5 (Lt,2H,Cl$CH_2$-); 2 (m,2H,-$CH_2$-C (ketal)-); 1.85 (m,2H,Cl-C$\underline{H_2}$); 1.5 (bs,1H,-OH); 1.35 (s,6H,-C($CH_3$)$_2$-).

f) [4-(1-ethylenedioxy-4-chlorobutyl)]α,α-dimethylacetic acid

14.4 g of substrate from the previous step (46 mmoles) were solubilized in acetone (1.015 l). The temperature was brought to 0 °C and Jones's reagent was added dropwise until the solution turns the colour of Jones's reagent. The operation required 30 ml of reagent.

The precipitate formed during the reaction was filtered by washing with ethyl acetate (EtOAc); the filtrate was diluted with 1 l of EtOAc and washed with water (5 x 300 ml) until obtaining a colourless aqueous phase. The organic phase was treated with an NaCl saturated solution and, after drying over anhydrous $Na_2SO_4$, was filtered and evaporated to dryness. 13 g of solid product were obtained. Yield 85%.

$^1$H-NMR ($CDCl_3$) δ: 10.08 (bs, -COOH); 7.35 (m,4H,-Ar-); 4-3.7 (2 symmetric m, 4H, -ketal-); 3.5 (t,2H,-Cl$CH_2$-); 2 (m,2H,-$CH_2$-C (ketal)-); 1.85 (m,2H,Cl-$CH_2$); 1.6 (s,6H,-C($CH_3$)$_2$-).

g) [4-(4-chlorobutyryl)]-phenyl-α,α-dimethylacetic acid chloride

4.5 g of the acid from the previous step were allowed to react with freshly distilled $SOCl_2$ (2 ml) and stirred in a $N_2$ environment at room temperature for 2 hours. The presence of the peak of COCl at 1790 $cm^{-1}$ was observed by I.R. analysis. The product was evaporated to dryness, optionally using anhydrous

toluene.

h) [4-(4-chlorobutyryl)]-phenyl-α,α-dimethylacetic acid amide

The product obtained according to the prevous reaction was taken up with anhydrous THF (2 to 3 ml) or anhydrous $Et_2O$ and added dropwise to a 30% $NH_3$ aqueous solution (31 ml) maintained at 0 °C. The mixture was stirred for approx. 20 min. and added with $H_2O$ (15 ml). A dark precipitate in crumbs was obtained, which was filtered and washed with $H_2O$. The filtrate was extracted with $CHCl_3$ (2 x 30 ml) and the solid was solubilized with $CHCl_3$.

The organic phases were combined, treated with brine and, after drying over $Na_2SO_4$, were filtered and evaporated to dryness. The dark oil obtained was triturated with hot petroleum ether. A brownish product was obtained (3.8 g), which was filtered and dried in a vacuum-pistol.

$^1$H-NMR ($CDCl_3$) δ: 8-7.5 (m,4H,Ar); 5.6-5.2 (2 bs, 2H, $-NH_2$); 3.7 (t,2H,Cl-$CH_2$-); 3.2 (t,2H,-$CH_2$-CO-); 2.2 (m,2H,Cl-$CH_2$-$CH_2$-); 1.6 (s,6H,-$C(CH_3)_2$-).

i) 4-(4-chlorobutyryl)-phenyl-α,α-dimethylacetonitrile

4.5 g of the amide from the previous step (16 mmoles) were reacted with 1.120 g of $Na_2CO_3$ and 2.1 ml of $POCl_3$ (25 mmoles) in 20 ml of anhydrous $CH_3CN$. The mixture was heated to reflux, stirred in an inert environment for 2.5 hours, filtered by washing with $CH_2Cl_2$, evaporated to dryness, taken up with $CH_2Cl_2$ (50 ml), washed with $H_2O$ (1 x 30 ml), treated with an $NaHCO_3$ saturated solution (1 x 30 ml), with brine (1 x 30 ml) and, after drying over $Na_2SO_4$ and filtration, evaporated to dryness. The raw product obtained was solubilized in THF (53 ml), added with conc. HCl (3.6 ml), stirred for 1.5 hour, added with $H_2O$ (200 ml), and extracted with EtOAc (3 x 100 ml). The combined organic phases were treated with brine and, after drying over $Na_2SO_4$, were concentrated under vacuum. The raw product obtained was purified by flash chromatography eluting with petroleum ether/EtOAc, 8:2.

2 g of product were obtained. Total yield of step g) through i) 46%.

$^1$H-NMR ($CDCl_3$) δ: 8-7.55 (m,4H,Ar); 3.7 (t, 2H, Cl-$CH_2$-); 3.2 (t,2H,-$CH_2$-CO-); 2.2 (m,2H,Cl-$CH_2$-$CH_2$-); 1.75 (s,6H,-$C(CH_3)_2$).

l) 4-(1-hydroxy-4-chlorobutyl)-phenyl-α,α-dimethylacetonitrile

A solution of the substrate from the previous step (1.9 g, 7 mmoles) in THF (150 ml), maintained at 0 °C, was added with 1.1 g of $NaBH_4$ solubilized in 70 ml of MeOH, allowed to stir for approx. 30 min., and neutralized with 10% HCl. After the solvent was evaporated to dryness, the mixture was taken up with EtOAc (200 ml), washed with $H_2O$ (2 x 150 ml), and treated with brine. After drying over $Na_2SO_4$, the solvent was evaporated to dryness. 2 g of product was obtained (quantitative yield).

IR ($CHCl_3$): sharp peak at 2250 $cm^{-1}$ (-CN).

$^1$H-NMR ($CDCl_3$) δ: 7.4 (m,4H,Ar); 4.7 (m, 1H, -CHOH-); 3.6 (m,2H,$ClCH_2$); 1.9 (m,4H,-$CH_2$-$CH_2$-CHOH); 1.75 (s,6H,-$C(CH_3)_2$-).

m) 4-[1-hydroxy-4(4-hydroxydiphenylmethyl-1-piperidinyl)-butyl-phenyl-α,α-dimethylacetonitrile

1,950 g of substrate from the previous step (7 mmoles) were allowed to react with 2 g of azacyclonol (7 mmoles), 2.6 g of anhydrous $K_2CO_3$ and 115 mg of KI, in 32 ml of anhydrous toluene and 8 ml of DMF. The mixture was heated to reflux and, after separation of the reaction water by Dean-Stark trap, was kept under stirring in an inert environment for 7 hours, and filtered. The filtrate was evaporated to dryness. The residue was taken up with $CHCl_3$ (300 ml), washed with $H_2O$ (3 x 150 ml), treated with brine, and, after drying over $Na_2SO_4$, filtered. The filtrate was evaporated to dryness. A raw product was obtained (6 g), which was purified by flash chromatography on a 4.5 cm silica gel column eluting with $CHCl_3$ and 2% $CHCl_3$/MeOH, 1:1.

2.1 g of product were obtained. Yield 60%.

IR ($CHCl_3$): 2250 $cm^{-1}$ (-CN).

$^1$H-NMR ($CDCl_3$) δ: 7.5-7 (m,14H,Ar); 4.55 (m, 1H, -CHOH-); 3 (m,2H); 2.5 (m,3H); 2.1-1.5 (m,1 OH), 1.7 (s,6H,-$C(CH_3)_2$-).

$^{13}$C-NMR ($CDCl_3$) δ: 146.155 (2 C quat Ar); 145.515, 139.410 (C quat Ar), 127.899, 126.134, 125.597, 124.560 (C arom); 109.017 (-CN); 79.037 (HO-$C(Ph)_2$-); 72.849 (-CHOH-); 58.579, 54.518, 53.156 ($CH_2$); 44.046 (4-CH-piperidinic); 39.617 ($CH_2$); 36.670 (-$C(CH_3)_2$-); 28.988 (-$C(CH_3)_2$-); 25.796 (2-$CH_2$-); 23.842 (-

$CH_2$-).

n) p-[1-hydroxy-4-(4-hydroxy-diphenylmethyl-1-piperidinyl) butyl]2-(1H-tetrazol-5-yl)-isopropylbenzene

A solution of substrate from the previous step (500 mg, 1 mmole) in anhydrous THF (2 ml) was fed to an autoclave, added with 2 ml of trimethylsilylazide (14 mmoles) and allowed to react for 24 hours at 120°C. The reaction mixture was concentrated under vacuum and the residue was refluxed in a 9:1 solution of MeOH/$H_2O$ (20 ml) for 3 hours under magnetic stirring.

The solvent was concentrated and the residue was purified by flash chromatography on silica eluting with $CHCl_3$/MeOH/hexane, 55:25:20. The product was further purified by medium-pressure chromatography on RP-8 eluting with MeOH/$H_2O$, 70:30.

100 mg of product were obtained. Yield 20%.

m.p. 156-157°C.

$^1$H-NMR ($CDCl_3$ + MeOD) $\delta$: 7.5-7 (m,14H,Ar); 4.4 (bm, 1H, -CH-OH-); 3.2 (bm,2H); 2.6 (bm,4H); 2-1.5 (m,OH), 1.7 (s,6H,-C($CH_3$)$_2$).

$^{13}$C-NMR ($CDCl_3$ + MeOH) $\delta$: 168 (C, quat, tetrazole); 147.204 (C quat Ar); 145.597 (2 C quat Ar); 142.069 (C quat Ar); 128.078, 126.387, 125.765, 125.489 (C Ar); 78.639 (HO-C(Ph)$_2$-); 72.299 (-CHOH-); 57.997, 54.030, 52.417 ($CH_2$); 42.022 (4'-CH-pyperidinic); 38.046 ($CH_2$); 36.146 (-C($CH_3$)$_2$-); 29.149 (-C-($CH_3$)$_2$-); 23.853, 21.095 ($CH_2$).

**EXAMPLE 3**

l) S-(-)-4-(1-hydroxy-4-chlorobutyl)-phenyl-$\alpha$,$\alpha$-dimethylacetonitrile
[compound of formula (XI) where Z = Cl, $R_2$ = H and $R_3$ = OH]

A solution of ketone of formula (X), where Z = Cl (6.20 g, 24.9 mmoles) in THF (100 ml) was added dropwise, over a period of 45 min., to a solution of (-)-B-chlorodiisopinocampheylborane [(-)-DIP-chloride] (11.9 g, 37.1 mmoles) in THF (110 ml), maintained at -25°C under magnetic stirring in an argon environment. Magnetic stirring was continued for 2 hours at - 25°C and for further 24 hours at room temperature. After solvent evaporation, the oily residue was dried overnight under high vacuum and dissolved again in ether (200 ml). The solution was added with diethanolamine (8.5 g, 80.8 mmoles) and the resulting mixture was stirred for 3 hours at room temperature. After filtration, the solid residue was washed with ether (30 ml) and with pentane (2 x 30 ml). The combined organic phases were evaporated to give a residue (11 g) which was purified by flash chromatography, eluting with petroleum ether-ethyl acetate, 8:2. The product under caption was obtained [compound of formula (XI), where Z = Cl, $R_2$ = H, $R_3$ = OH] (5.4 g, 87%), m.p. 79°C to 80°C;

$^1$H-NMR ($CDCl_3$) $\delta$: 1.75 (3H,s,-$CMe_2$), 1.90 (4H,m,***CH**$_2$* ***CH**$_2$*CHOH), 2.20 (1H, br s [broad singlet], OH), 3.55 (2H,t,J = 6Hz, $CH_2Cl$), 4.72 (1H, br s, ***CH***OH), 7.40 (4H 2 xd, J = 8Hz,Ar); $[\alpha]^{20}_D$ = -28.3 (c = 1, $CHCl_3$).

m) S-(-)-4-[1-hydroxy-4-(4-hydroxydiphenylmethyl-1-piperidinyl)-butyl]phenyl-$\alpha$,$\alpha$-dimethylacetonitrile [compound of formula (XII) where $R_2$ = H and $R_3$ = OH]

$\alpha$,$\alpha$-Diphenyl-4-piperidyl-methanol (azacyclonol, 5.6 g, 20.8 mmoles), anhydrous potassium carbonate (7.28 g, 52.7 mmoles) and potassium iodide (0.312 g, 1.88 mmoles) were added to a solution of the compound of formula (XI), where Z = Cl, $R_2$ = H and $R_3$ = OH (5.2 g, 20.8 mmoles) in toluene (130 ml) and DMF (21 ml). The resulting mixture was refluxed for 8 hours in an argon environment, while the reaction water was eliminated by Dean-Stark trap. The reaction mixture was cooled and filtered. After solvent evaporation, the residue was dissolved again in chloroform (400 ml), washed with water (3 x 200 ml), with a sodium chloride saturated solution (200 ml), and dried over anhydrous sodium sulphate. After solvent evaporation, the residue (10 g) was purified by flash chromatography with dichloromethane containing from 2 to 5% methanol, to give the compound of formula (XII), where $R_2$ = H and $R_3$ = OH (4.5 g, 45%), m.p. 174 - 176°C;

$^1$H-NMR ($CDCl_3$) $\delta$: 1.40-2.20 (15H,m,2xMe, 4x$CH_2$, ***CH***$CPh_2OH$), 2.50 (4H,m,$CH_2$-N-$CH_2$), 3.05 (2H, 2xd, J = 12Hz, $CH_2$***CH**$_2$*N), 4.60 (1H,m,***CH***OH), 7.10-7.55 (14H,m,aromatic protons); $[\alpha]^{20}_D$ = -53.4 (c = 2, $CHCl_3$).

n) S-(-)-4-[1-hydroxy-4-(4-hydroxydiphenylmethyl-1-piperidinyl)-butyl]-1'-(tetrazol-5-yl)isopropylbenzene [derivative of formula (I) where $R_1$ = H,$R_2$ = H and $R_3$ = OH]

Azidotrimethylsilane (1.736 g, 15.07 mmoles) was added to a solution of derivative of formula (XII), where $R_2$ = H and $R_3$ = OH (0.52 g, 1.20 mmoles) in THF (3 ml). The resulting mixture was heated to 120 °C in an autoclave for 36 hours. After cooling and solvent evaporation, the residue (0.57 g) was dissolved in THF (9 ml) and added with tetrabutylammonium fluoride (0.480 g, 1.8 mmoles). The resulting mixture was stirred overnight at 40 °C, diluted with chloroform (80 ml), washed with water (5 x 30 ml), and dried over anhydrous sodium sulphate. After solvent evaporation, the residue was purified by flash chromatography eluting with chloroform containing from 3 to 20% methanol to give the derivative of formula (I), where $R_1$ = H, $R_2$ = H and $R_3$ = OH (0.100 g, 16%), which was further purified by medium-pressure reversed phase chromatography eluting with methanol-water 7:3.

$^1$H-NMR ($CDCl_3$) $\delta$: 1.40-1.90 (15H,m,2xMe, 4x$CH_2$, ***CH***$CPh_2OH$), 2.58 (4H,m,$CH_2$-N-$CH_2$), 3.20 (2H,m,$CH_2$ ***$CH_2$***N), 4.36 (1H,m,***CH***OH), 6.98-7.40 (14H,m,aromatic protons);
$^{13}$C-NMR ($CDCl_3$ + $CD_3OD$) $\delta$: 20.8, 23.9, 28.8, 36.0, 38.0, 42.2, 52.6, 52.9, 56.8, 72,3 (CHOH), 78.4 ($PH_2$***C***-OH), 77.3, 125.4, 125.7, 126.4, 128.0, 142.0. 145.5, 146.6, 167.0 ($Me_2$C-C = N).

**EXAMPLE 4**

l) R-( + )-4-(1-hydroxy-4-chlorobutyl)-phenyl-α,α-dimethylacetonitrile [compound of formula (XI) where Z = Cl, $R_2$ = OH and $R_3$ = H]

A solution of ketone of formula (X), where Z = Cl (1.0 g, 4.0 mmoles) in THF (10 ml) was added dropwise, over a period of 20 min., to a solution of ( + )-B-chlorodiisopinocampheylborane [( + )-DIP-chloride] (1.93 g, 6.0 mmoles) in THF (21 ml), maintained under magnetic stirring in an argon environment at -25 °C for 30 min. Magnetic stirring was continued for further 48 hours at room temperature. After solvent evaporation, the oily residue was dried overnight under high vacuum and dissolved again in ether (50 ml). The solution was added with diethanolamine (1.37 g, 13.04 mmoles) and the resulting mixture was stirred for 3 hours at room temperature. After filtration, the solid residue was washed with ether (10 ml) and with pentane (2 x 10 ml). The combined organic phases were evaporated to give a residue (3 g) which was purified by flash chromatography, eluting with petroleum ether-ethyl acetate, 8:2. The compound of formula (XI), where Z = Cl, $R_2$ = OH, $R_3$ = H] was obtained (0.70 g, 70%); $[\alpha]^{20}_D$ = + 27.5 (c = 1, $CHCl_3$).

m) R-( + )-4-[1-hydroxy-4-(4-hydroxydiphenylmethyl-1-piperidinyl)-butyl]phenyl-α,α-dimethylacetonitrile [compound of formula (XII) where $R_2$ = OH and $R_3$ = H]

α,α-Diphenyl-4-piperidyl-methanol (azacyclonol, 0.648 g, 2.28 mmoles), anhydrous potassium carbonate (0.840 g, 6.08 mmoles) and potassium iodide (0.036 g, 0.217 mmoles) were added to a solution of the compound of formula (XI), where Z = Cl, $R_2$ = OH and $R_3$ = H, (0.570 g, 2.28 mmoles) in toluene (20 ml) and DMF (2.58 ml). The resulting mixture was refluxed for 9 hours in an argon environment, while the reaction water was removed by means of a Dean-Stark trap. The reaction mixture was cooled and filtered.

After solvent evaporation, the residue was dissolved again in chloroform (50 ml), washed with water (3 x 20 ml), with a sodium chloride saturated solution (20 ml), and dried over anhydrous sodium sulphate. After solvent evaporation, the residue (3 g) was purified by flash chromatography with dichloromethane containing from 2 to 5% methanol, to give the compound of formula (XII), where $R_2$ = OH and $R_3$ = H (0.490 g, 45%); $[\alpha]^{20}_D$ = + 50.2 (c = 2, $CHCl_3$).

n) R-( + )-4-[1-hydroxy-4-(4-hydroxydiphenylmethyl-1-piperidinyl)-butyl]-1'-(tetrazol-5-yl)isopropylbenzene [derivative of formula (I) where $R_1$ = H,$R_2$ = OH and $R_3$ = H]

Azidotrimethylsilane (1.736 g, 15.07 mmoles) was added to a solution of the compound of formula (XII), where $R_2$ = OH and $R_3$ = H, (0.49 g, 1.0 mmoles) in THF (3 ml). The resulting mixture was heated to 120 °C in an autoclave for 48 hours. After cooling and evaporating the solvent, the residue was dissolved in THF (9 ml) and added with tetrabutylammonium fluoride (0.370 g, 1.17 mmoles). The resulting mixture was stirred overnight at 40 °C, diluted with chloroform (80 ml), washed with water (5 x 30 ml), and dried over anhydrous sodium sulphate. After solvent evaporation, the residue was purified by flash chromatography eluting with chloroform containing from 3 to 20% methanol to give the derivative of formula (I), where $R_1$ = H, $R_2$ = OH and $R_3$ = H (0.024 g, 4.6%), which was further purified by medium-pressure reversed phase chromatography eluting with methanol-water, 7:3.

**PHARMACOLOGICAL TESTS**

The following abbreviations have been used:
TER = terfenadine
TFZ = terfetrazole = p-1-hydroxy-4-(4-hydroxydiphenylmethyl-1-piperidinyl)-butyl-2-(1H-tetrazol-5-yl)-isopropyl benzene
MET I = 4-(1-hydroxy)-4-(4-hydroxydiphenylmethyl)-1-piperidinyl)-butyl-$\alpha,\alpha$-dimethylphenylacetic acid (metabolite of TER)

**MATERIALS AND METHODS**

The tested compouds (TER, TFZ and MET I) were in racemic form.
Compound TFZ was prepared as described in Example 2.

**1) Assay of receptor binding towards histaminergic $H_1$ receptor.**

**1a) Competitive displacement assays**

Guinea pigs weighing 200 to 500 g were used to evaluate the molecule affinity for the $H_1$ receptors, expressed on the cerebral cortex according to the method described by R.M. Wallace and J.M. Young, Mol. Pharmacol., **23**, 60 1983. In brief, the cerebral cortex was suspended in a buffer (50 mM potassium phosphate, pH 7.5), homogenized with Polytron, and centrifuged (35,000 rpm) for 10 min. at 4°C. The resulting pellet was washed by centrifugation (35,000 rpm for 30 min.) and the membranes were suspended in a buffer (600 $\mu$g/ml proteins) and used for the assay, according to the method described by R. Chang, V. Tran, and S. Synder, Eur. J. Pharmacol., **48**, 463, 1978. Briefly, the membranes were incubated with ($^3$H)-mepyramine (specific activity 31.2 Ci/mM) at a concentration of $10^{-9}$M for 30 min. at 25°C. Bound radioactivity was separated from free radioactivity by centrifuging (maximum speed microcentrifuge). Non-specific binding was determined in the presence of 200 $\mu$g promethazine.

Promethazine displaces the mepyramine bound to the $H_1$ receptors through specific binding. The residual binding is the non-specific binding, resulting from the difference between total and specific bindings. $IC_{50}$ values of the molecules being examined (i.e. the concentration values inhibiting the radioligand linkage by 50%) were determined from the binding competition curves; the $K_i$ values (corresponding to the molar concentrations at which the receptor semisaturation was obtained) were calculated, as described by Y.C. Cheng, W.H. Prusoff, Biochem. Pharmacol., **22**, 3099, 1973, by the following equation:

$$K_i = \frac{IC_{50}}{1 + \frac{[^3h\text{-mepyramine}]}{K_D}}$$

using a $K_D$ value of ($^3$H)mepyramine equal to 0.8 nM (found experimentally). The reported results are means ± S.E.M. of 3 to 6 experiments, each being carried out in triplicate. Proteins were determined by the technique developed by Smith et al. (P.K. Smith, R.I. Krohn, G.T. Hermanson, A.K. Mallia, F.H. Gartner, M.D. Provenzano, E.K. Fujimoto, N.M. Goche, B.J. Olson, D.C. Klenk, Anal. Biochem., **150**, 77, 1985) with bicinchoninic acid.

A second series of tests was carried out, according to the method described by S.J. Hill, J.M. Young, and D.H. Marrian, Nature (London), **270**, 361, 1977, with peripheral tissue (guinea pig ileum) as a source of $H_1$ receptors, the guinea pig terminal ileum longitudinal musculature being known to express the $H_1$ receptors.

**Results and discussion**

A first series of experiments, in which radiolabelled receptors from guinea pig cerebral cortex were tested with increasing concentrations of MET I, TFZ or TER, was carried out to verify the possibility of a typical competitive displacement of said molecules in respect of the labelled ligand. The data obtained are recapitulated in Fig. 1, where the straight lines representing the percentage of receptors occupied by the drugs under examination are reported as a function of increasing concentrations, according to a logarithmic scale.

The analysis of said straight lines, which represent the binding capacity of the drugs under examination to the $H_1$ receptor, shows an entirely superimposable trend for MET I and TFZ. This finding is in support of an absolutely comparable binding affinity of the two molecules for the $H_1$ receptors. This is also proved by the corresponding $IC_{50}$ values obtained from the two straight lines by graphical interpolation (Table 1).

Therefore, TFZ possesses a satisfactory displacement capability in respect of the high affinity radioligand, wholly equivalent to that of MET I, which is a well known metabolite of TER and whose antihistaminic anti-$H_1$ activity is already known (C. & EN, 14 June, 1993).

Furthermore, Fig. 1 shows that TER, the parental molecule of MET I, when compared with the two molecules described above, possesses a higher ability to displace the labelled ligand from the $H_1$ receptors: this may be assumed from the position of the straight line representing said property, which is on the left of the two straight lines relating to the other tested compounds, though extending in a parallel direction. This is a very important finding, the characteristic of parallel trend being highly in favour of a colon pharmaco-dynamic mechanism.

Fig. 2 is a graphical representation of the results obtained from a series of experiments analogous to the previous ones, with the sole exception that the receptors were obtained from guinea pig ileum. Said results show that, also for the $H_1$ receptors obtained from peripheral tissue, TFZ shows a good affinity level, comparable with that of MET I.

Also in this case, the parental molecule TER shows quite a higher affinity: this may be inferred from a comparative evaluation between the corresponding values of $IC_{50}$ (obtained by graphical interpolation) and of $K_i$ (calculated as described under "Materials and Methods") (Table 1).

Table 1

$IC_{50}$ [M] and corresponding $K_i$ values, obtained by radioligand competitive displacement assay carried out with the tested drugs

| Drug | Type of tissue | | | |
|---|---|---|---|---|
| | Cerebral cortex | | Ileum | |
| | $IC_{50}$ | $K_i$ | $IC_{50}$ | $K_i$ |
| TER | $7x10^{-9}$ | $2.43x10^{-9}$ | $8x10^{-10}$ | $2.70x10^{-10}$ |
| MET I | $5x10^{-8}$ | $1.74x10^{-8}$ | $5x10^{-8}$ | $1.74x10^{-8}$ |
| TFZ | $8x10^{-8}$ | $2.78x10^{-8}$ | $3x10^{-7}$ | $1.04x10^{-8}$ |

$IC_{50}$ : concentrations inhibiting the radioligand linkage by 50%

$K_i$ : molar concentrations at which receptor semisaturation is obtained

The relation between $IC_{50}$ and $K_i$ is defined by the aforementioned equation.

**1b - Binding assays under saturation kinetics conditions**

These assays (Scatchard) were carried out under the same incubation conditions and according to the conclusive test method described under paragraph 1a), with the exception that the radioligand concentration was changed according to a range of concentrations comprised between 0.3 and 10 nM, in the presence of a concentration of antagonist TFZ of $2 \times 10^{-7}$ M.

Scatchard's diagram shows the value of $K_D$ and $B_{max}$ (maximal binding sites of the receptor system).

**Results and discussion**

With a view to evaluating the competitive nature of the TFZ binding to the $H_1$ receptor, the affinity and possible saturation of ligand ($^3H$)mepyramine were estimated as a moiety of free ligand concentration in the presence of 2 x $10^{-7}$ M TZF. The results obtained (Fig. 3) show that, in the presence of TFZ, no significant change in the density of the binding sites takes place ($B_{max}$ is determined by the intersection of the straight line with the X axis), whereas a change in $K_D$ of ($^3H$) mepyramine takes place in the presence of TFZ, which becomes equal to $9.1x10^{-9}$ M, as calculated from the straight line slope. This datum is comparable with the values calculated by the competitive displacement assays reported in Table 1.

Therefore, this datum supports the existence of a single high-affinity TFZ binding site. The TFZ bond with said site is competitive.

**2) Evaluation of the functional activity of the tested molecules on guinea pig ileum**

The test was essentially meant to characterize the type of activity of the molecules under examination by checking their antagonist activity versus $H_1$ receptor. To this end, the guinea pig isolated ileum was used for two different tests: a) evaluation of the antagonist activity towards the agonist histamine; b) evaluation of the antagonist activity towards the agonist acetylcholine. Said tests were carried out to check the specificity and selectivity levels of the moleculas under examination.

Male and female guinea pigs weighing approx. 250 g were used. A portion of terminal ileum was excised and immersed in a bath for isolated organ, in Tyrode solution, at 37 °C. The antagonist activity was evaluated by determining the ability of the studied drug to modify the dose-effect curve built with increasing doses of agonist alone. Furthermore, in another series of experiments, increasing doses of the molecules under examination were tested in comparison with a submaximal dose of agonist.

The dose of histamine used was $6.8x10^{-8}$ M. The percentage of inhibition found after adding the tested drug to the bath for isolated organ versus the response obtained from the agonist alone was calculated. Six detections were carried out for each drug concentration. The results, calculated in terms of $IC_{50}$, corresponded to the drug concentrations responsible for a 50% reduction in the submaximal concentration of the agonist alone.

**Results and discussion**

The characterization of the functional activity of molecule TFZ performed with guinea pig isolated ileum showed that the molecule under examination had no agonist activity, being incapable of inducing contraction of the ileum smooth muscles when added at increasing doses to the bath perfusion liquid.

The evaluation of the antagonist activity towards different agonists provided the following evidences.

**A) Activity of TFZ on histamine-induced contractions**

Fig. 4 is a graphical representation of the results obtained by assaying the effects of different TFZ concentrations on the response to histamine.

These effects were estimated according to the double reciprocal calculation (reciprocal of the effect observed versus the reciprocal of the dose used), as proposed by H. Lineweaver and D. Burk, J. Am. Chem. Soc., 56, 658 (1934). As known, in a graph of this type, a common point of the regression straight lines intersecting the ordinates axis is indicative of a competitive antagonism. Therefore, the results obtained by this type of investigation are in agreement with the results obtained through the binding test with radioligands already described.

A subsequent series of tests was carried out with a view to determining the $IC_{50}$ values of TFZ and MET I and, therefore, performing a comparative evaluation of the activity. To this end, a histamine concentration capable of inducing a submaximal contraction (6.8 x $10^{-8}$ M) was tested in the presence of increasing concentrations of the studied drugs. The results are shown in Fig. 5, which is a graphical representation of the % inhibition obtained versus increasing doses of MET I (Fig. 5 A) or of TFZ (Fig. 5 B). The corresponding values of $IC_{50}$ reported in the figure may be obtained by graphical interpolation. The results obtained suggest that the antihistaminic activity characterizing molecule TFZ, evaluated through this type of a functional test, is completely superimposable to that of MET I, the $IC_{50}$ values being not significantly different.

### B) TFZ activity towards acetylcholine-induced contractions

With a view to estimating the antagonist specificity level of the molecule TFZ under examination and taking into account the anticholinergic activity often associated with antihistaminic molecules, the capability of TFZ of antagonizing the acetylcholine (ACh) contraction effect on guinea pig isolated ileum was tested. Therefore, a dose-effect curve was built testing increasing ACh doses in respect of a TFZ dose corresponding to the $IC_{50}$ effective against histamine. The results obtained (Fig. 6) clearly show that TFZ possesses no inhibitory activity against said agonist.

## CONCLUSIONS

The following conclusions may be drawn from the aforementioned results.

1) Molecule TFZ proved to possess high affinity for the $H_1$ receptor expressed on guinea pig cerebral cortex or ileum. This statement is supported by the results obtained through tests with radioligands, both in competitive displacement assays (Figs. 1 and 2) and in binding tests under saturation kinetics conditions (Fig. 3).

2) Comparative evaluation of the affinity level of TFZ towards for the $H_1$ receptor in respect of terfenadine metabolite (MET I) provided evidence that the two molecules are characterized by a wholly comparable affinity level (see Table 1).

3) Compared with the two compounds previously described, terfenadine molecule (TER) proved to possess a quite higher affinity (Table 1).

4) Evaluation of the functional activity of molecule TFZ proved that the tested compound possesses a competitive antagonist activity towards histamine (Fig. 4), wholly comparable with that of compound MET I (Figs. 5A and 5B).

5) The antagonist activity of TFZ is specific towards $H_1$ receptor, while it has not be evidenced towards the muscarinic cholinergic receptor at doses active towards $H_1$ (Fig. 6).

## Claims

**1.** A terfenadine derivative of formula (I)

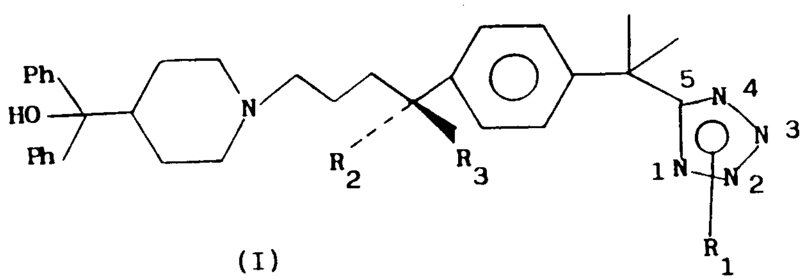

(I)

where $R_1$, which is in position 1 or 2 of the tetrazole ring, stands for H, or a linear or branched alkyl group containing from 1 to 6 carbon atoms; $R_2$ and $R_3$, different from one another, are selected between H and OH, and the pharmaceutically acceptable salts thereof.

**2.** The derivative of formula (I) according to claim 1, wherein $R_1$ is H, said derivative being selected from the group consisting of enantiomer S (where $R_2 = H$ and $R_3 = OH$), enantiomer R (where $R_2 = OH$ and $R_3 = H$), and any mixture thereof.

**3.** The derivative of formula (I) according to claim 2, as a racemic mixture.

**4.** A Process for the preparation of a derivative of formula (I)

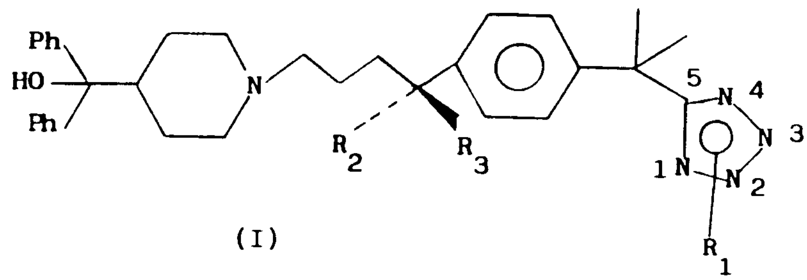

(I)

where $R_1$, which is in position 1 or 2 of the tetrazole ring, stands for H, or a linear or branched alkyl group containing from 1 to 6 carbon atoms; $R_2$ and $R_3$, different from one another, are selected between H and OH, as racemic mixtures or as single enantiomers, comprising the following steps:

a) reducting $\alpha,\alpha$-dimethylphenylacetic acid to give 2-methyl-2-phenylpropanol;

b) protecting of the alcohol group of 2-methyl-2-phenylpropanol to give the compound of formula (II)

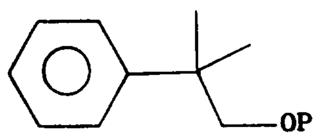

(II)

where P is a protective group of the alcoholic moiety which is stable under the successive acylation conditions;

c) acylating aromatic ring of the compound of formula (II) by reacting it with the acyl halide of formula (III)

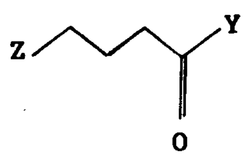

(III)

where Y and Z, identical or different, are halogens, to give the ketone of formula (IV)

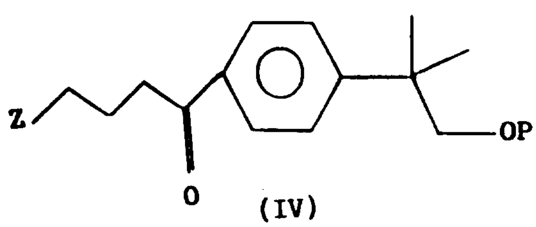

(IV)

where P and Z are as defined above;

d) protecting of the ketone moiety of the aforesaid compound of formula (IV) by reaction with a hydroxylated compound, to give the ketal of formula (V)

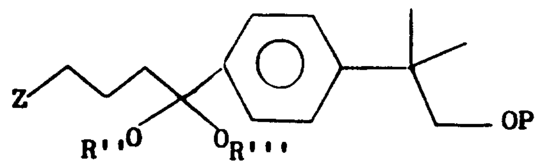

(V)

where P and Z are as defined above, R'' and R''', identical or different, are each linear or branched alkyl groups containing from 1 to 6 carbon atoms, or taken together, stand for a linear or branched aliphatic residue which, together with the oxygen atoms to which it is bound, forms a heterocyclic ring of from 5 to 7 atoms;

e) deprotecting the alcoholic moiety of the product obtained under d) to give the product of formula (VI)

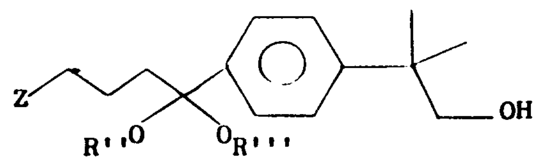

(VI)

where Z, R'' and R''' are as defined above;

f) oxidazing the alcoholic moiety of the aforesaid product of formula (VI) to give the carboxylic acid of formula (VII)

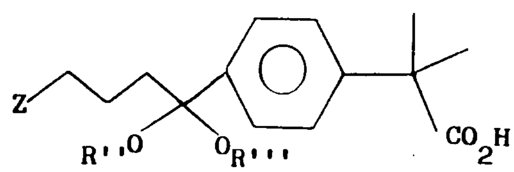

(VII)

where Z, R'' and R''' are as defined above;

g) converting the carboxylic acid of formula (VII) into the acylic halide of formula (VIII)

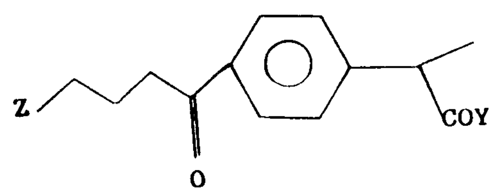

(VIII)

where Z and Y are as defined above;

h) treating the compound of formula (VIII) with ammonia to give the corresponding amide of formula (IX)

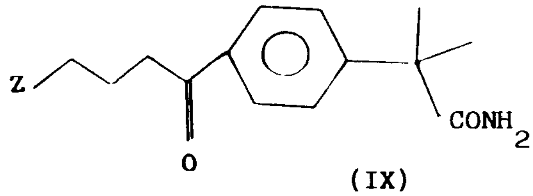

(IX)

where Z is as defined above;

i) converting the amide of formula (IX) into the nitrile of formula (X)

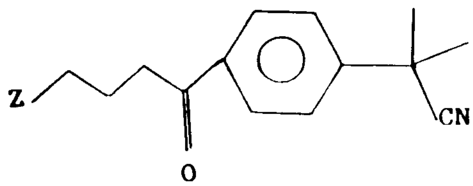

(X)

where Z is as defined above;

l) reducing the ketone moiety of the compound of formula (X) to give the alcohol derivative of formula (XI)

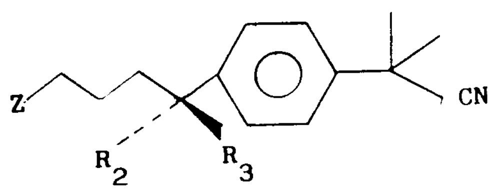

(XI)

where $R_2$, $R_3$ and Z are as defined above, to obtain, when using an optically inactive reducing agent, the derivative of formula (XI) in racemic form, which is then converted, by steps m) through o), into the corresponding derivative of formula (I), which is also in racemic form, or to obtain, when using an optically active reducing agent such as B-chlorodiisopinocampheyl borane, the derivatives of formula (XI) in optically active form; in particular, enantiomer S is obtained when using the levorotatory form (-) of said borane and enantiomer R when using the dextrorotatory form (+) of said borane, forms S and R of the derivative of formula (XI) being respectively converted into forms S and R of derivatives of formula (I) by steps m) through o) of the present process;

m) condensing the derivative of formula (XI) with azacyclonol to give the compound of formula (XII)

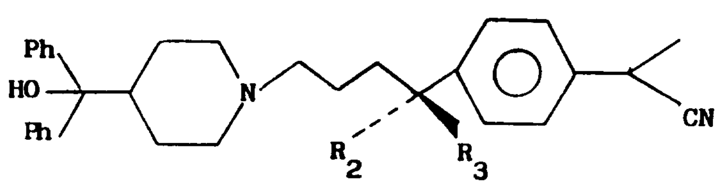

(XII)

where $R_2$ and $R_3$ are as defined above;

n) forming the tetrazole ring by reacting the cyano-derivative of formula (XII) with an azide to give the derivative of formula (I) where $R_1$ is H;

o) optionally, alkylating the tetrazole ring with an alkylating agent able to transfer linear or branched alkyl groups containing from 1 to 6 carbon atoms, to give the derivative of formula (I), where $R_1$ is a linear of branched alkyl containing from 1 to 6 carbon atoms.

**5.** The process according to claim 4, wherein:

a) is carried out with a reducing agent of the carboxylic moiety, in an anhydrous aprotic solvent, at 0 °C to 40 °C;

in step b), P is an acyl group, introduced by reaction with an acylating agent selected from the group consisting of an acyl halide and an acyl anhydride, at 0 °C to 50 °C, optionally in the presence of an organic solvent, and in the presence of an organic base;

c) is carried out with a Lewis acid, in an inert organic solvent, at - 20 °C to +20 °C;

in d) the hydroxylated compound is a diol, whose aliphatic residue, either linear or branched, contains from 2 to 12 carbon atoms, and the reaction is carried out in an anhydrous aprotic solvent, in the presence of an acid catalyst, at +50 °C to +100 °C;

in e) the derivative of formula (V) is treated with a strong inorganic base, in an alcoholic solvent, at 0 °C to +50 °C;

f) is carried out with Jones's reagent, at neutral pH, at -20 °C to +20 °C, in an organic solvent that is inert under the oxidation conditions;

g) is carried out with a halogenating agent, in excess in respect of the starting compound; and operating at 0 °C to 40 °C, in an anhydrous medium, optionally in the presence of an organic aprotic solvent,

h) is carried out at -20 °C to +20 °C, in an aqueous medium;

in step i), the amide of formula (IX) is treated with a dehydrating agent, in an anhydrous aprotic solvent, in the presence of a base, at +40 °C to +100 °C; then, the raw product recovered from the reaction mixture is treated with a strong acid, in an aprotic solvent, at 0 °C to +40 °C;

l) is carried out with sodium borohydride, in an anhydrous organic solvent, at -20 °C to +20 °C, or with optically active B-chlorodiisopinocampheyl borane, in an anhydrous organic solvent, at -40 °C to 0 °C; the borane intermediate obtained is hydrolyzed by treatment with a hydroxylated compound at +20 °C to +30 °C;

step m) is carried out in the presence of an organic or inorganic base, and of a catalytic amount of an alkaline iodide, working in an anhydrous medium, in an organic aprotic solvent, at +80 °C to +150 °C;

step n) is carried out with an azide selected from the group consisting of trimethylsilylazide, tributyltinazide and sodium azide, in an anhydrous medium, in an organic aprotic solvent selected among ether solvents, aromatic solvents, and mixtures thereof, at +15 °C to +180 °C, and if necessary is followed by a chemical treatment to convert of the intermediates formed into tetrazole derivatives of formula (I), where $R_1$ = H;

step o) is carried out with an alkylating agent $R^1$-W, where $R^1$ is a linear or branched alkyl group containing from 1 to 6 carbon atoms and W is a halogen, in the presence of a base, in an anhydrous organic solvent.

**6.** The process according to claim 4, wherein:

in a) the reducing agent is $LiAlH_4$ and the solvent is an ether;

in b) P is acetyl, the organic solvent is a halogenated solvent, the organic base is a tertiary alkylamine containing from 3 to 20 carbon atoms, a heterocyclic aromatic base or a mixture thereof;

in c) the Lewis acid is $AlCl_3$, the organic solvent is an aromatic solvent deactivated towards aromatic eletrophilic substitutions, a halogenated solvent or $CS_2$;

in d) the diol is ethylene glycol, the acid catalyst is p-toluenesulphonic acid, trifluoroacetic acid or gaseous HCl, the solvent is benzene or toluene, the reaction is carried out separating the water formed by azeotropic distillation;

in e) the inorganic base is an alkaline hydroxide;

in g) the halogenating agent is thionyl chloride or thionyl bromide;

in l) the solvent is an alcohol solvent, an ether solvent or a mixture thereof;

in step m) the base is an alkaline or alkaline earth carbonate or bicarbonate, the solvent is a slightly polar solvent, a strongly polar solvent or a mixture thereof;

step n) is carried out according to one of the conditions described below:

- trimethylsilylazide in tetrahydrofuran, in autoclave, at 100 ° C to 140 ° C;
- trimethylsilylazide, in the presence of diethylazodicarboxylate and $Ph_3P$, in tetrahydrofuran, at room temperature;
- tributyltinazide in toluene, at reflux temperature;
- sodium azide, in the presence of ammoniun chloride, in dimethylformamide, at 110 ° C to 120 ° C:
- sodium azide, in the presence of $Et_3N.HCl$ and of 1-methyl-2-pyrrolidinone, at 150 ° C;

furthermore, when -trimethylsilylazide is used, the product recovered from the reaction mixture is treated with aqueous alcohols;
in step o) W is Br, the base is an alkaline alkoxide in an alcohol solvent, or an alkaline hydride in an alcohol solvent or in an ether solvent.

**7.** The process according to claim 5, wherein:
in a), the solvent is ethyl ether and the reaction temperature is 20 ° C to 30 ° C;
in b) the acylating agent is acetic anhydride, the organic base is a mixture of triethylamine and 4-pyrrolidinepyridine, the organic solvent is methylene chloride, the reaction temperature is 15 ° C to 30 ° C;
c) is carried out using the product of formula (II), where P is acetyl, and the acyl halide of formula (III), where Y = Z = Cl; the Lewis acid is $AlCl_3$, the solvent is $CS_2$, the temperature is 0 ° C to 5 ° C; the derivative of formula (III) is added to an $AlCl_3$ suspension, in excess in respect of the acyl halide (III); then the substrate of formula (II) is added;
d) is carried out using the compound of formula (IV) where Z = Cl and P = acetyl; the solvent is benzene, the acid catalyst is p-toluenesulphonic acid, the reaction temperature is the reflux temperature of the reaction mixture;
e) is carried out using derivative of formula (V) where Z = Cl, P = acetyl; and R'' and R''', taken together, stand for $-(CH_2)_2$, the alkaline hydroxide is NaOH used in excess, the alcohol solvent is methanol, the temperature is 15 ° C to 30 ° C;
f) is carried out on the derivative of formula (VI) where Z = Cl and R'' and R''', taken together, stand for $-(CH_2)_2$; the solvent is acetone and Jones's reagent is added to the derivative of formula (VII) until the reaction mixture turns the colour of Jones's reagent, at 0 to + 5 ° C;
g) is carried out on the compound of formula (VII) where Z = Cl and R'' and R''', taken together, stand for $-(CH_2)_2$, the halogenating agent is thionyl chloride, used in the absence of organic solvents, and in an excess of 1.5 to 2 times as moles in respect of the substrate to be halogenated; the reaction temperature is 15 ° C to 30 ° C;
step h) is carried out on the derivative of formula (VIII) where Y = Z = Cl, additing a solution of said derivative in an anhydrous ether solvent selected between anhydrous ethyl ether and anhydrous tetrahydrofuran, to aqueous ammonia in stoichiometric excess in respect of the product of formula (VIII), at 0 ° C;
i) is carried out on the amide of formula (IX) where Z = Cl, the dehydrating agent is $POCl_3$, the base is $Na_2CO_3$, the anhydrous aprotic solvent is acetonitrile and the treatment with the dehydrating agent is carried out at the reflux temperature of the reaction mixture; in the subsequent treatment with acids, the acid is concentrated hydrochloric acid, the aprotic solvent is tetrahydrofuran, and the temperature is 15 ° C to 30 ° C;
in step l) when sodium borohydride is used, the compound of formula (X) has Z = Cl, the solvent is a mixture of methanol and tetrahydrofuran, the temperature is 0 ° C to + 5 ° C; when optically active B-chlorodiisopinocampheyl borane is used, the compound of formula (X) where Z = Cl is used, the reduction is carried out in anhydrous tetrahydrofuran,
at -25 ° C for about 2 hours, then the reaction is maintained at room temperature for a further period of about 24 to 48 hours, and the resulting intermediate borate is hydrolyzed by treatment with diethanolamine, in anhydrous ethyl ether, at room temperature to give the corresponding derivative of formula (XI);
step m) is carried out on the derivative of formula (XI) where Z = Cl, the alkaline iodide is KI, the base is anhydrous potassium carbonate, the solvent is a mixture of toluene and dimethylformamide, the temperature is the reflux temperature of the reaction mixture;
step n) is carried out with trimethylsilylazide, in tetrahydrofuran, in autoclave at + 100 ° C to + 140 ° C; the product recovered after solvent evaporation is treated with methanol/$H_2O$ at reflux or, alternatively, with a quaternary ammonium fluoride in ether solvent, at + 20 ° C to + 60 ° C;
in step o) the base is $MeO^-Na^+$ and the solvent is methanol.

**8.** Therapeutic compositions containing as active ingredient an effective dose of at least one terfenadine derivative of formula (I)

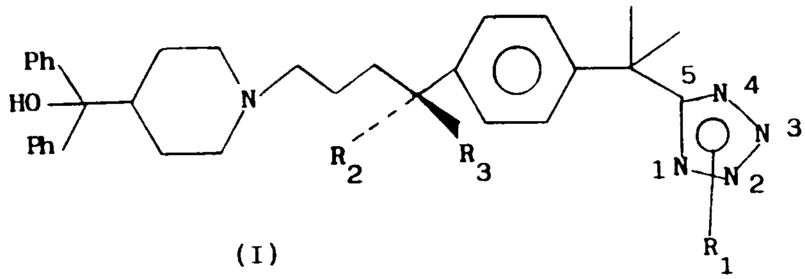

(I)

where $R_1$, which is in position 1 or 2 of the tetrazole ring, is H, or a linear or branched alkyl group containing from 1 to 6 carbon atoms; $R_2$ and $R_3$, different from one another, are H or OH, and the pharmaceutically acceptable salts thereof.

**9.** The therapeutic compositions according to claim 8, useful in human therapy for the treatment of the diseases for which histamine is held to be responsible.

**10.** The therapeutic compositions according to claim 9, wherein said diseases are allergic diseases and ulcerative conditions of the gastrointestinal apparatus.

**11.** The therapeutic compositions according to claim 10, wherein the allergic diseases are seasonal allergic rhinitides or asthma.

**12.** The therapeutic compositions according to claim 8, suitable for oral administration.

**13.** The therapeutic compositions according to claim 8, suitable for parenteral administration.

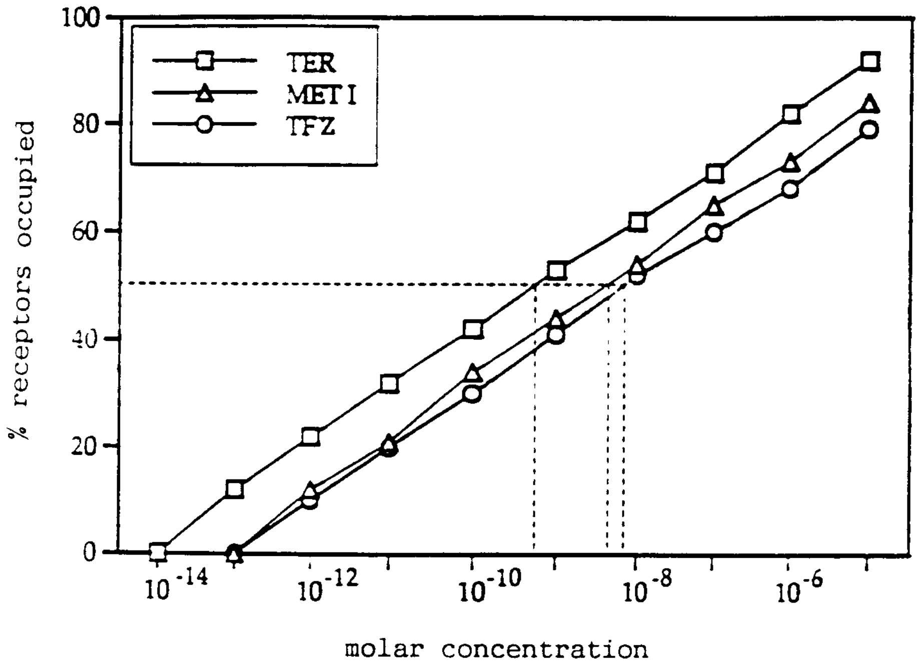
100
80
60
40
20
0
% receptors occupied
TER
MET I
TFZ
$10^{-14}$
$10^{-12}$
$10^{-10}$
$10^{-8}$
$10^{-6}$
molar concentration

*Fig.1*

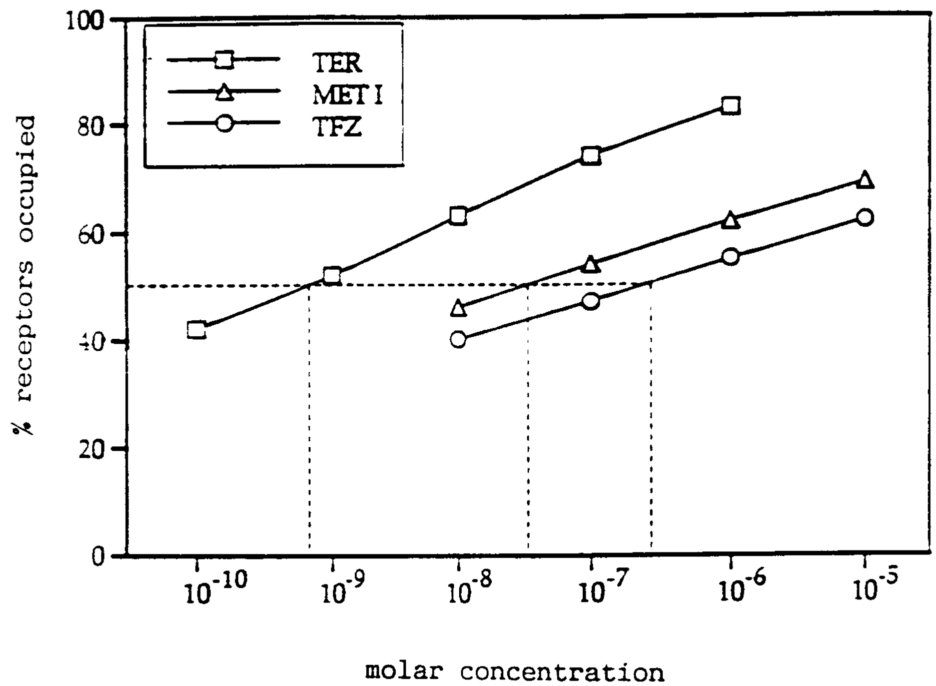
100
80
60
40
20
0
% receptors occupied
TER
MET I
TFZ
$10^{-10}$
$10^{-9}$
$10^{-8}$
$10^{-7}$
$10^{-6}$
$10^{-5}$
molar concentration

*Fig.2*

*Fig. 3*

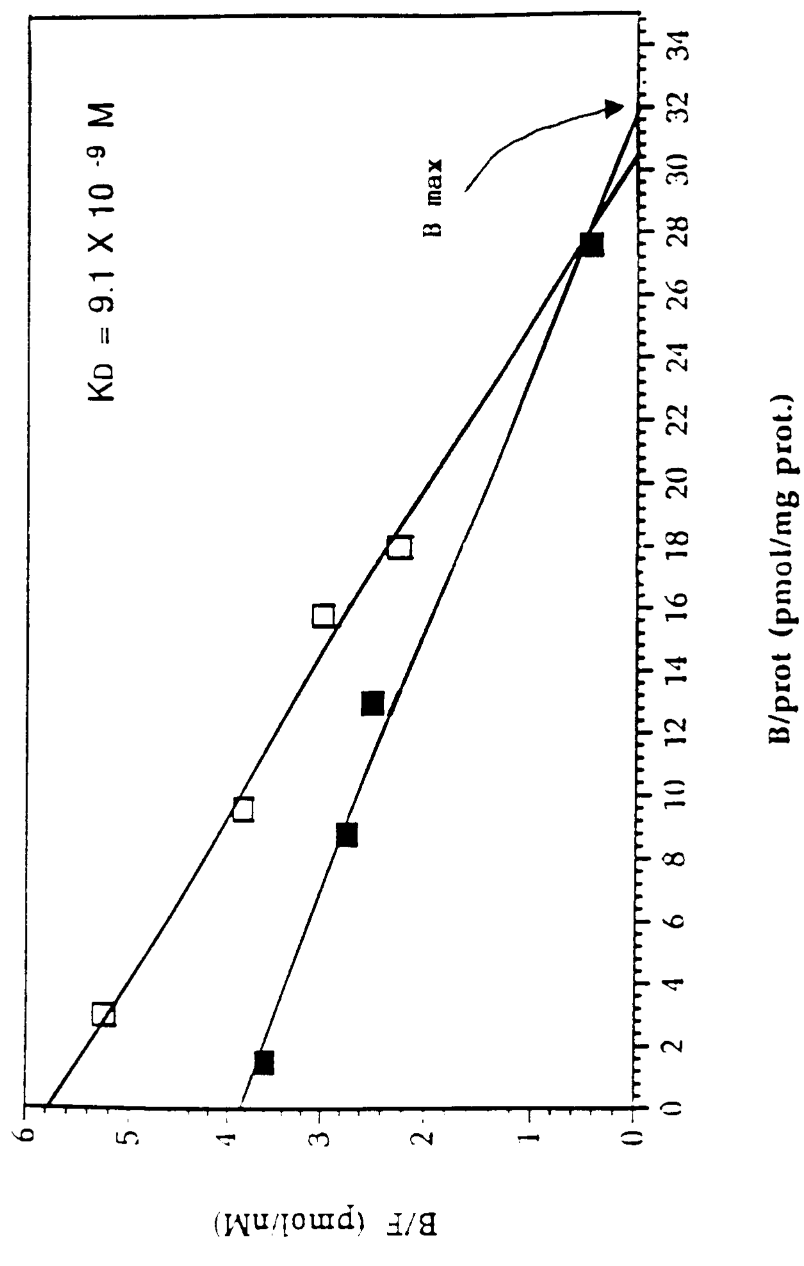
KD = 9.1 X 10 -9 M
B max
B/F (pmol/nM)
B/prot (pmol/mg prot.)
0
1
2
3
4
5
6
0
2
4
6
8
10
12
14
16
18
20
22
24
26
28
30
32
34

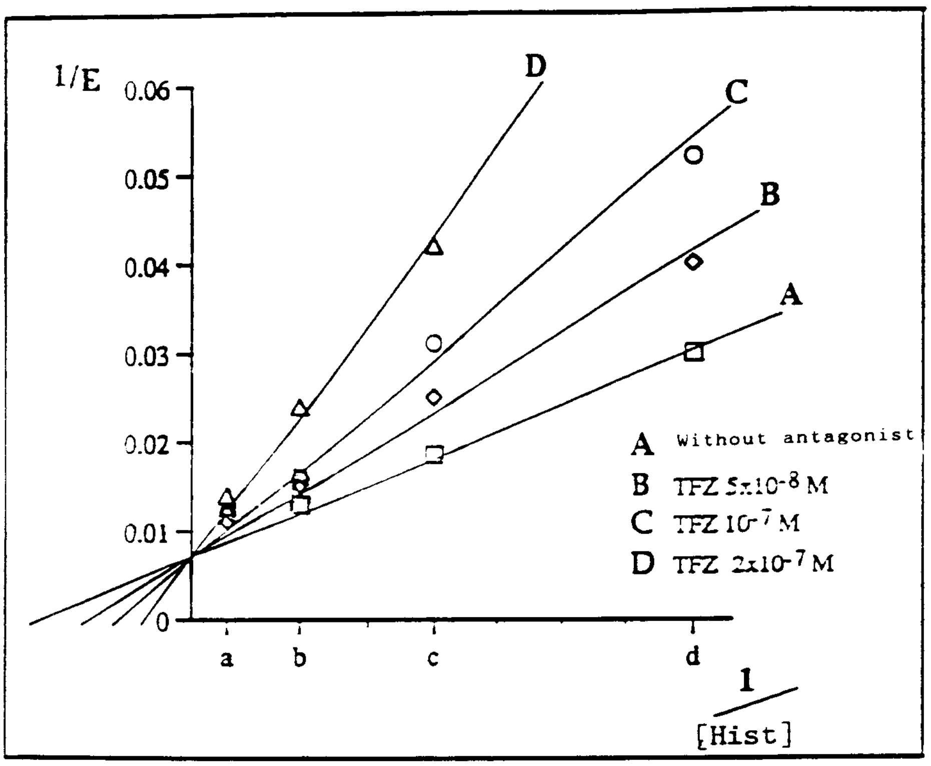
1/E
0.06
0.05
0.04
0.03
0.02
0.01
0
a
b
c
d
D
C
B
A
A Without antagonist
B TFZ 5x10-8 M
C TFZ 10-7 M
D TFZ 2x10-7 M
1
[Hist]

*Fig.4*

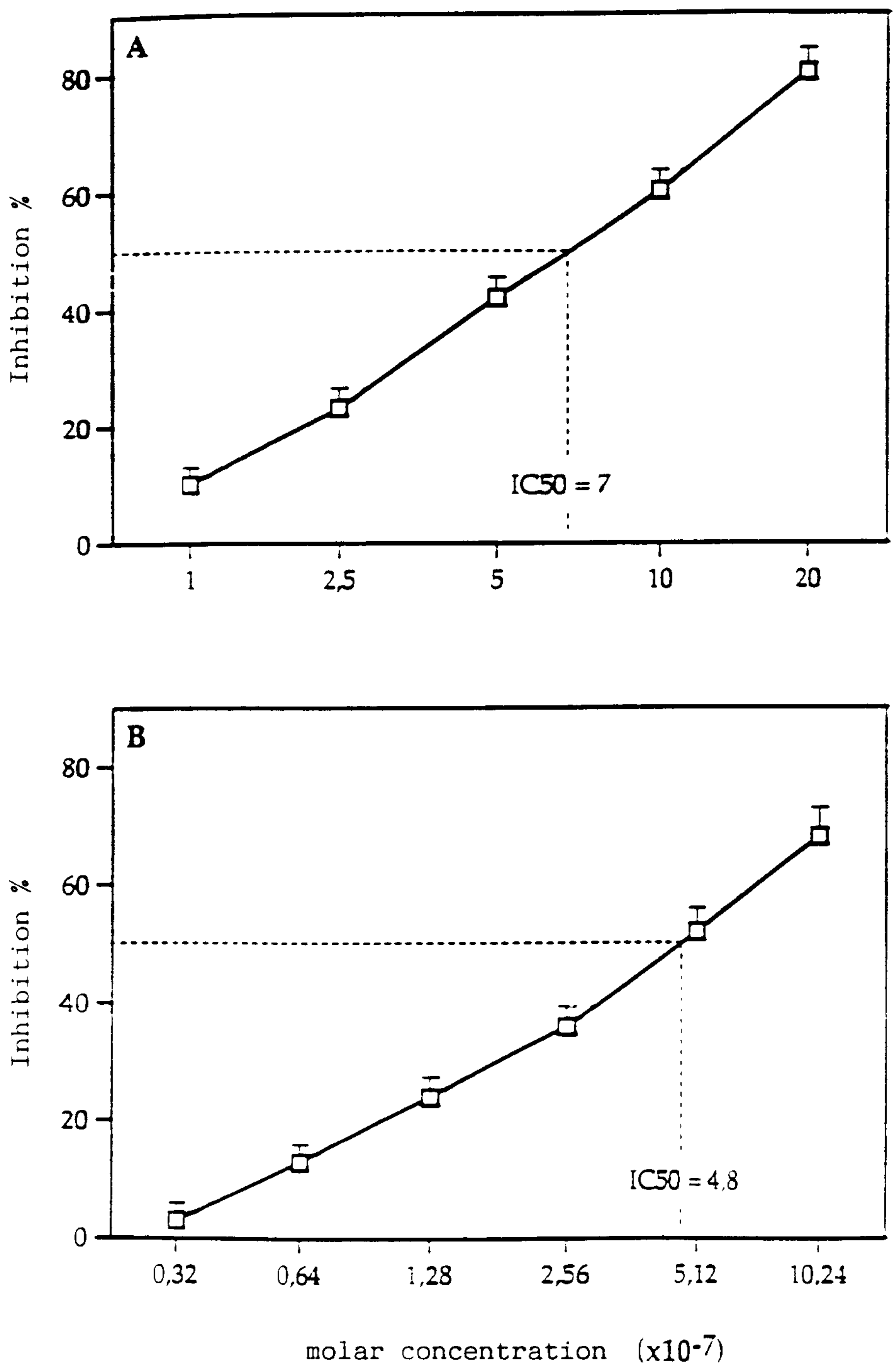
A
Inhibition %
80
60
40
20
0
IC50 = 7
1
2,5
5
10
20
B
Inhibition %
80
60
40
20
0
IC50 = 4,8
0,32
0,64
1,28
2,56
5,12
10,24
molar concentration (x10-7)

*Fig. 5*

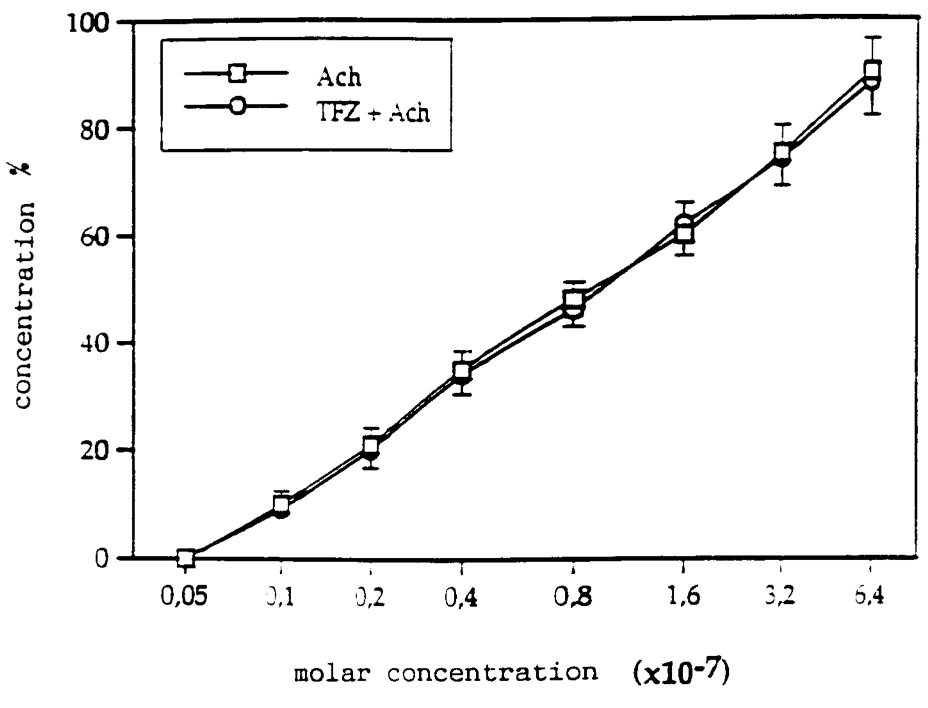
100
80
60
40
20
0
concentration %
Ach
TFZ + Ach
0,05
0,1
0,2
0,4
0,8
1,6
3,2
6,4
molar concentration (x10-7)

*Fig.6*

European Patent Office

# EUROPEAN SEARCH REPORT

Application Number
EP 94 11 5981

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,Y | WO-A-93 23047 (MERRELL DOW) 25 November 1993<br>* the whole document *<br>* claim 1 * | 1-13 | C07D401/10<br>A61K31/445 |
| Y | JOURNAL OF MEDICINAL CHEMISTRY.,<br>vol.10, 1967, WASHINGTON US<br>pages 149 - 154<br>see page 149, right column, lines 8-18 | 1-13 | |
| A | ARZNEIMITTELFORSCHUNG/DRUG RESEARCH,<br>vol.32, 1982<br>pages 1185 - 1190<br>* the whole document * | 1-13 | |
| Y | PHARMACEUTICAL RESEARCH COMMUNICATIONS,<br>vol.15, no.2, February 1983<br>pages 119 - 129<br>* page 120, line 15 - page 121 * | 1-13 | |
| P,A | J. ORG. CHEM.,<br>vol.59, 1994<br>pages 2620 - 2622<br>* the whole document * | 1-13 | TECHNICAL FIELDS SEARCHED (Int.Cl.6)<br>C07D |

The present search report has been drawn up for all claims

| Place of search | Date of completion of the search | Examiner |
|---|---|---|
| THE HAGUE | 2 February 1995 | Kissler, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)